# EUROPEAN PATENT APPLICATION

(11) **EP 4 306 131 A1**
(43) Date of publication of application: **17.01.2024**
(21) Application number: 22766215.2
(22) Date of filing: 03.03.2022
(51) Int. Cl.: A61K 48/00, C12N 15/864, C12N 15/861, A01N 63/00

(54) **DELIVERY OF ANTIBODY BY USING DUAL VIRAL VECTOR SYSTEM**

(30) Priority: 08.03.2021 CN 202110252231
(71) Applicant: Nanjing GenScript Biotech Co., Ltd., Nanjing, Jiangsu 211100 (CN)
(72) Inventor: WANG, Weiming, Nanjing, Jiangsu 211100 (CN); ZHANG, Xian, Nanjing, Jiangsu 211100 (CN); MI, Yanling, Nanjing, Jiangsu 211100 (CN); LI, Zhongdao, Nanjing, Jiangsu 211100 (CN); SHAO, Cuiying, Nanjing, Jiangsu 211100 (CN)
(74) Representative: Pharma Patents International AG
(86) International application number: PCT/CN2022/079047
(87) International publication number: WO 2022/188689

(57) **Abstract**

Provided is a dual recombinant viral vector system for expressing, in a cell, a polypeptide molecule which is composed of two different peptide chains and has an antigen binding activity, wherein the first chain is expressed using a first viral vector, the second chain is expressed using a second viral vector, and the first and second chains can be assembled, after being expressed in a cell, into the polypeptide molecule which has an antigen binding activity. Further provided are a dual plasmid system for the production of the dual recombinant viral vector system, a host cell containing the dual recombinant viral vector system, a pharmaceutical composition, and the use of and the method for delivering the polypeptide molecule to a subject by using the dual recombinant viral vector system.

## Description

### Cross Reference to Related Application

The present application claims the right of priority for the Chinese invention patent application with the filing date being 08 March 2021 and the application number being 202110252231.1, which is incorporated herein by reference in its entirety.

### Technical Field

The present invention relates to the field of gene therapy, particularly relates to a system and method for expressing an antibody by using a dual viral vector system.

### Background Art

In recent years, recombinant adeno-associated virus (rAAV) has been shown to be capable of delivering genomic components to different tissues of various species and to various types of cells, showing that this new therapy has good prospects. The rAAV drug Alipogene tiparvovec (Glybera, uniQure), approved by the European Medicines Agency (EMA) in 2012, consists of a rAAV1 vector and a human lipoprotein lipase (LPL) gene, which is used for treating lipoprotein lipase deficiency (LPLD). So far, two other rAAV drugs have been approved by the FDA and EMA, namely voretigene neparvovec-rzyl (Luxturna, Spark) and Onasemnogene abeparvovec (ZOLGENSMA, AveXis/Novartis), the latter being conditionally approved, both of which are rAAV-mediated complete gene replacement therapies. Some clinical gene therapy trials using rAAV vectors to deliver different genomic components use the mechanisms of action that have been shown to be effective in animal models, including: truncated genes, such as B-domain deleted factor VIII genes for treating haemophilia and micro-dystrophin for treating DMD; small RNA, such as small hairpin RNA for treating hepatitis B, microRNA for treating Huntington's disease, and small nuclear RNA that induces exon skipping and is used for treating DMD; and expressed antibodies for treating AMD.

Antibody drugs have been shown to be safe and effective in a wide range of indications. rAAV is an ideal vector for the delivery of antibody genes, and the length of monoclonal antibody genes falls within the packaging capacity of rAAV. At present, the rAAV-based antibody delivery strategies use single rAAV vectors to express the polypeptides of light and heavy chains of an antibody in a transduced cell and allow the self-assembly of IgG by self-cleaving peptides (such as different 2A). However, such strategies have a low efficiency in the expression of antibodies and may lead to non-functional antibodies or antibody protein aggregates due to incorrect assembly of antibodies, which can cause toxic effects, limiting the clinical use thereof. So far, the only rAAV-based IgG drug undergoing clinical trials is AAV8-VRC07, which expresses a HIV-1 neutralizing antibody and is used in antiviral therapies. Other similar strategies include expressing a soluble antibody without Fc fragments (RGX-314) or recombinant proteins fused with Fc fragments (AAV2-sFLT01 and ADVM-022).

Other viral vectors can also be used for delivering genomic components, but also suffer from low antibody expression efficiency and low assembly efficiency. There is currently a lack of general methods for efficient antibody expression in vivo.

### Summary of the Invention

The present invention provides a method for efficiently expressing a polypeptide molecule having an antigen binding activity in vivo by using recombinant viral vectors, wherein nucleic acid sequences encoding two polypeptide chains constituting the polypeptide molecule are placed in two expression cassettes, respectively, and are inserted between 5' inverted repeat sequences and 3' inverted repeat sequences of two recombinant viral vectors, respectively. The expression cassettes separately express the two polypeptide chains constituting the polypeptide molecule antibody in a host cell, and the two polypeptide chains are assembled into a polypeptide molecule having an antigen binding activity.

The first aspect of the present invention provides a dual recombinant viral vector system for expressing, in a cell, a polypeptide molecule which is composed of two different peptide chains and has an antigen binding activity, comprising:
a first recombinant viral vector containing, in a genome thereof, a first expression cassette for expressing the first chain of the polypeptide molecule, and
a second recombinant viral vector containing, in a genome thereof, a second expression cassette for expressing the second chain of the polypeptide molecule,
wherein the first chain and the second chain are different; the first recombinant viral vector and the second recombinant viral vector, after being introduced into a cell, can express the first chain and the second chain, respectively, and the first and second chains can be assembled into a polypeptide molecule having an antigen binding activity.

In some embodiments, the first recombinant viral vector and the second recombinant viral vector are independently selected from the group consisting of a recombinant adeno-associated viral vector, a recombinant adenoviral vector, a recombinant HSV vector, a recombinant lentiviral vector and a recombinant retroviral vector.

In some embodiments, the first recombinant viral vector and the second recombinant viral vector are recombinant adeno-associated viral vectors, or the first recombinant viral vector and the second recombinant viral vector are lentiviral vectors.

In some embodiments, the first recombinant viral vector and the second recombinant viral vector are recombinant adeno-associated viral vectors, and respectively contain a capsid of AAV1, AAV2, AAV3, AAV4, AAV5, AAV6, AAV7, AAV8, AAV9, AAV10, AAV11, AAV12, AAV13 or AAV Rh10, or a chimeric AAV capsid or a modified capsid.

In some embodiments, the first recombinant viral vector and the second recombinant viral vector contain an ITR of AAV1, AAV2, AAV3, AAV4, AAV5, AAV6, AAV7, AAV8, AAV9, AAV10, AAV11, AAV12, AAV13 or AAV Rh10.

In some embodiments, the polypeptide molecule contains an antigen binding moiety having a heavy chain variable region and a light chain variable region, the first chain contains the heavy chain variable region of the antigen binding moiety, and the second chain contains the light chain variable region of the antigen binding moiety.

In some embodiments, the polypeptide molecule contains an antigen binding moiety having a heavy chain and a light chain, the first chain contains the heavy chain of the antigen binding moiety, and the second chain contains the light chain of the antigen binding moiety.

In some embodiments, a ratio of the first recombinant viral vector to the second recombinant viral vector is 4 : 1-1 : 8, preferably 1 : 1-1 : 4.

In some embodiments, the polypeptide molecule is a monoclonal antibody or an antigen binding fragment thereof, the first chain is a heavy chain of the monoclonal antibody or the antigen binding fragment thereof, and the second chain is a light chain of the monoclonal antibody or the antigen binding fragment thereof.

In some embodiments, the antigen binding fragment of the monoclonal antibody is an Fab fragment, an Fab' fragment or a (Fab')₂ fragment.

In some embodiments, the monoclonal antibody or the antigen binding fragment thereof specifically binds to an angiogenic factor.

In some embodiments, the angiogenic factor is selected from VEGF, VEGFR, FGF, PDGF, TGFβ, Ang1, Ang2, integrin, CD31 and a plasminogen activator.

In some embodiments, the polypeptide molecule is a bispecific antibody comprising a first antigen binding moiety that specifically binds to a first antigenic epitope and a second antigen binding moiety that specifically binds to a second antigenic epitope, the first antigenic epitope and the second antigenic epitope are different, the first antigen binding moiety comprises a heavy chain and a light chain, the first chain contains the heavy chain of the first antigen binding moiety, and the second chain contains the light chain of the first antigen binding moiety.

In some embodiments, the first antigen binding moiety in the bispecific antibody is a monoclonal antibody or an antigen binding fragment thereof, preferably the antigen binding fragment is an Fab fragment, an Fab' fragment or a (Fab')₂ fragment.

In some embodiments, the second antigen binding moiety in the bispecific antibody is an antibody in the form of a single chain.

In some embodiments, the antibody in the form of a single chain is a single domain antibody or scFv.

In some embodiments, the second antigen binding moiety is linked to the N-terminus or C-terminus of the heavy chain of the first antigen binding moiety; and the first chain contains the heavy chain of the first antigen binding moiety, and the second antigen binding moiety.

In some embodiments, the second antigen binding moiety is linked to the N-terminus or C-terminus of the light chain of the first antigen binding moiety; and the second chain contains the light chain of the first antigen binding moiety, and the second antigen binding moiety.

In some embodiments, the first chain contains the heavy chain of the first antigen binding moiety, and the second antigen binding moiety; and the second chain contains the light chain of the first antigen binding moiety.

In some embodiments, at least one or both of the first antigen binding moiety and the second antigen binding moiety specifically bind to an angiogenic factor.

In some embodiments, the angiogenic factor is selected from VEGF, VEGFR, FGF, PDGF, TGFβ, Ang1, Ang2, integrin, CD31 and a plasminogen activator.

In some embodiments, the first antigen binding moiety specifically binds to VEGF, and the second antigen binding moiety specifically binds to Ang2; or the first antigen binding moiety specifically binds to Ang2, and the second antigen binding moiety specifically binds to VEGF.

In some embodiments, the polypeptide molecule contains a first antigen binding moiety that specifically binds to a first antigenic epitope and a second binding domain that specifically binds to a second target molecule, the second target molecule is a cytokine, the first antigen binding moiety comprises a heavy chain and a light chain, the first chain contains the heavy chain of the first antigen binding moiety, and the second chain contains the light chain of the first antigen binding moiety.

In some embodiments, the second binding domain is a cytokine receptor, an extracellular domain thereof, or their variants.

In some embodiments, the first antigen binding moiety is a monoclonal antibody or an antigen binding fragment thereof, preferably the antigen binding fragment is an Fab fragment, an Fab' fragment or a (Fab')₂ fragment.

In some embodiments, the second binding domain is linked to the N-terminus or C-terminus of the heavy chain of the first antigen binding moiety; and the first chain contains the heavy chain of the first antigen binding moiety, and the second binding domain.

In some embodiments, the second binding domain is linked to the N-terminus or C-terminus of the light chain of the first antigen binding moiety; and the second chain contains the light chain of the first antigen binding moiety, and the second binding domain.

In some embodiments, the first chain contains the heavy chain of the first antigen binding moiety, and the second binding domain; and the second chain contains the light chain of the first antigen binding moiety.

In some embodiments, the first antigen binding moiety specifically targets and binds to PD-1, PD-L1 or an angiogenic factor, and the second binding domain is a receptor or a fragment thereof capable of binding to human transforming growth factor β (TGFβ), Ang2 or VEGF.

In some embodiments, the angiogenic factor is selected from VEGF, VEGFR, FGF, PDGF, TGFβ, Ang1, Ang2, integrin, CD31 and a plasminogen activator.

In some embodiments, the first antigen binding moiety specifically binds to PD-L1, and the second binding domain is a human transforming growth factor β receptor II (TGFβRII) or a fragment thereof.

In some embodiments, the dual viral vector system is a composition containing the first recombinant viral vector and the second recombinant viral vector.

A second aspect of the present invention provides a dual plasmid system for producing any one of the foregoing dual recombinant viral vector systems, comprising:
a first plasmid containing the first expression cassette and a viral genomic element required for the production of the first recombinant viral vector; and
a second plasmid containing the second expression cassette and a viral genomic element required for the production of the second recombinant viral vector.

A third aspect of the present invention provides a host cell transduced by using any one of the foregoing dual recombinant viral vector systems.

A fourth aspect of the present invention provides a pharmaceutical composition containing any one of the foregoing dual recombinant viral vector systems or the foregoing host cell, and a pharmaceutically acceptable carrier.

A fifth aspect of the present invention provides the use of any one of the foregoing dual recombinant viral vector systems, the foregoing dual plasmid system or the foregoing host cell in the preparation of a polypeptide molecule which is composed of two different peptide chains and has an antigen binding activity.

A sixth aspect of the present invention provides a method for producing a polypeptide molecule which is composed of two different peptide chains and has an antigen binding activity, comprising the following steps:
transducing a host cell by using any one of the foregoing dual recombinant viral vector systems;
and culturing the transduced host cell under conditions that enable the first chain and second chain of the polypeptide molecule to be expressed in the host cell and assembled to produce a polypeptide molecule having an antigen binding activity, thereby obtaining the polypeptide molecule.

A seventh aspect of the present invention provides the use of any one of the foregoing dual recombinant viral vector systems in the preparation of a drug for delivering a polypeptide molecule which is composed of two different peptide chains and has an antigen binding activity to a subject.

An eighth aspect of the present invention provides a method for delivering a polypeptide molecule which is composed of two different peptide chains and has an antigen binding activity to a subject, comprising: administering to a subject in need thereof any one of the foregoing dual recombinant viral vector systems or the foregoing pharmaceutical composition.

A ninth aspect of the present invention provides any one of the foregoing dual recombinant viral vector systems or the foregoing pharmaceutical composition, for use in delivering a polypeptide molecule which is composed of two different peptide chains and has an antigen binding activity to a subject.

### Brief Description of the Drawings

The present invention will be more fully understood from the following detailed description in combination with the accompanying drawings, in which like elements are numbered alike, wherein:

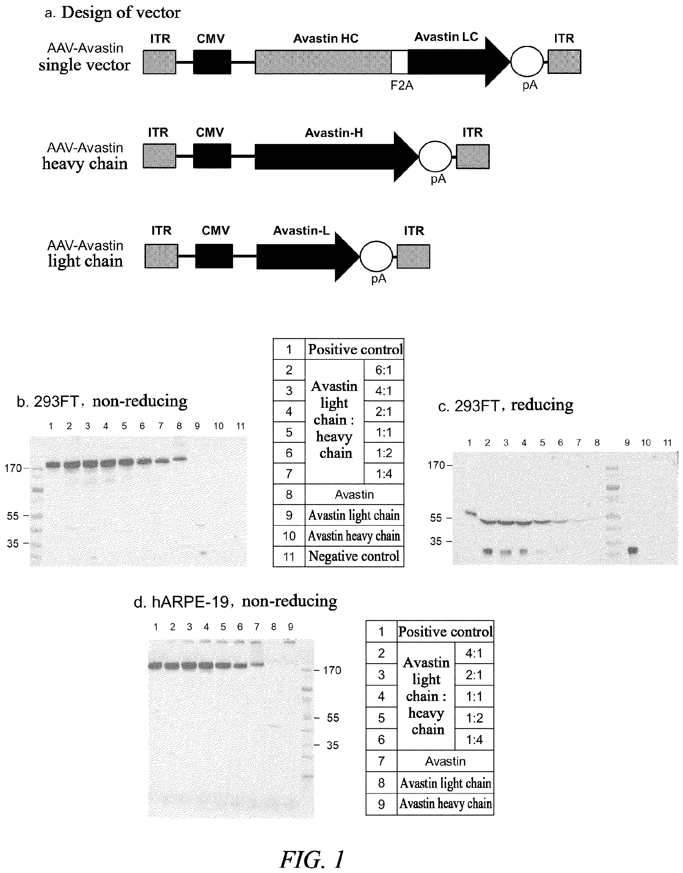

FIG. 1 shows the expression of Avastin by using dual AAV vectors. Diagram a shows schematic diagrams of a vector expressing a complete antibody sequence (an AAV-Avastin single vector), a vector expressing a heavy chain only (an AAV-Avastin heavy chain, i.e., AAV-Avastin-H), and a vector expressing a light chain only (and an AAV-Avastin light chain, i.e., AAV-Avastin-L). Diagrams b and c show the Western blot results of Avastin produced in HEK293FT cells transduced with a single vector (AAV2-Avastin) and dual vectors (AAV2-Avastin-L and AAV2-Avastin-H), respectively, in which diagram b is under a non-reducing condition, diagram c is under a reducing condition, the positive control is a purified antibody, and the negative control is a cell culture supernatant not transduced with a recombinant viral vector. Diagram d shows the Western blot results of Avastin produced in hARPE-19 cells transduced with a single vector (AAV2-Avastin) and dual vectors (AAV2-Avastin-L and AAV2-Avastin-H), respectively, in which the positive control is a purified antibody.
FIG. 2 shows the Western blot results of Avastin expressed in HEK293FT cells or hARPE-19 cells transduced with a single vector or dual vectors having an AAV8 capsid (diagram a), an AAV9 capsid (diagram b) and an AAV5 capsid (diagram c), in which the positive control is a purified antibody.
FIG. 3 shows the Western blot results of Avastin expressed in HEK293FT cells or hARPE-19 cells transduced with dual AAV vectors and transfected with dual plasmid vectors, in which the positive control is a purified antibody.
FIG. 4 shows the Western blot results of Avastin expressed in hiPSC-RPE cells transduced with dual AAV vectors, in which the positive control is a purified antibody.

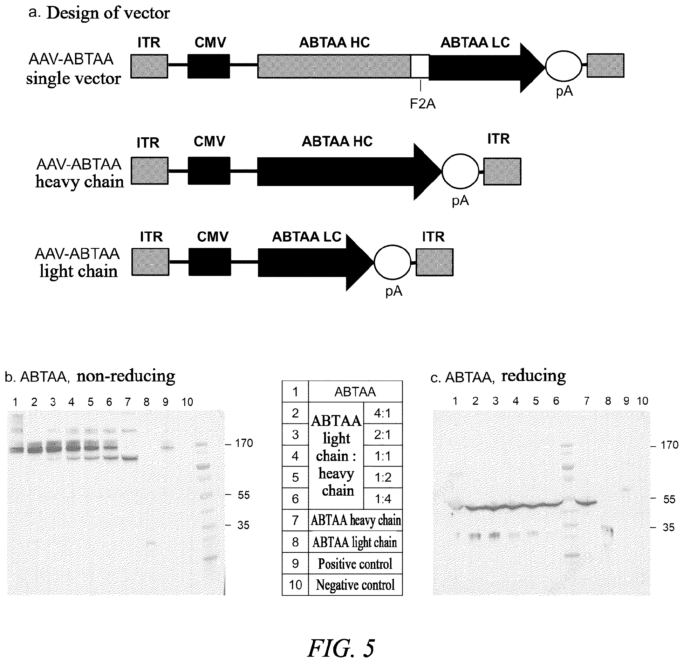

FIG. 5 shows the expression of ABTAA by using dual AAV vectors. Diagram a shows schematic diagrams of a transfer plasmid expressing a complete antibody sequence (pAAV2.CMV.ABTAA) and transfer plasmids expressing only a light chain or a heavy chain (pAAV2.CMV.ABTAA-L and pAAV2.CMV.ABTAA-H). Diagrams b and c show the Western blot results of ABTAA expressed in HEK293FT cells transduced with a single vector or dual AAV vectors, in which diagram b is under a non-reducing condition, diagram c is under a reducing condition, the positive control is a purified antibody, and the negative control is a cell culture supernatant not transduced with a recombinant viral vector.

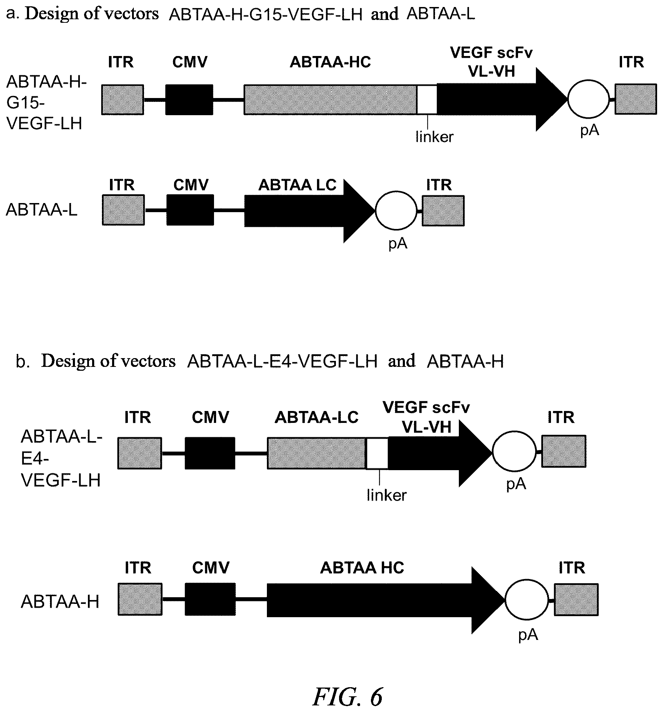

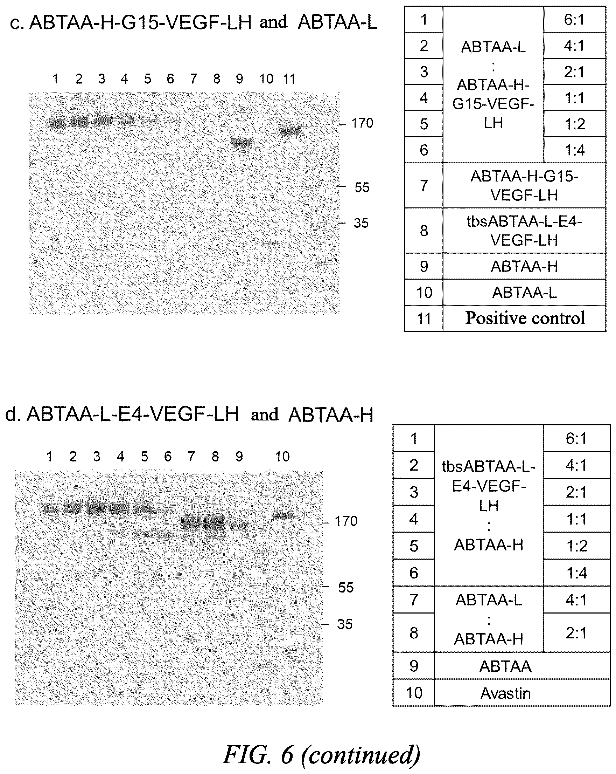

FIG. 6 shows the expression of a bispecific antibody against Ang2 and VEGF by using dual AAV vectors. Diagrams a and b show schematic diagrams of the dual AAV vectors for expressing the bispecific antibody, in which as shown in diagram a, the variable region of anti-VEGF antibody is linked to the heavy chain of ABTAA, and as shown in diagram b, the variable region of anti-VEGF antibody is linked to the light chain of ABTAA. Diagrams c and d respectively show the Western blot results of an ABTAA-anti-VEGF bispecific antibody expressed in HEK293FT cells transduced with the dual viral vectors respectively as shown in diagrams a and b, in which the positive control is a purified antibody.
FIG. 7 shows the expression of Avastin gene delivered by dual AAV vectors in mice.

### Detailed Description of Embodiments

The present invention provides a dual recombinant viral vector system for delivering an antibody component and the use thereof, wherein using two recombinant viral vectors to respectively deliver the two chains of the antibody component can increase the expression of the antibody component, particularly using two recombinant viral vectors to respectively deliver the heavy chain and light chain of the antibody component can promote the efficient assembly of the heavy chain and light chain of the antibody component, increase the pairing efficiency of the heavy chain and light chain, and reduce inaccurate pairing.

Taking recombinant AAV vectors as an example, the dual recombinant viral vector system of the present invention shows advantageous application effects. In the existing design of using rAAV vectors for antibody delivery, ORFs of heavy and light chains are packaged into the same rAAV vector. Due to the size limitation of gene packaging in rAAV, it is difficult to use rAAV to deliver antibodies that exceed the packaging capacity of rAAV (such as bispecific antibodies). In addition, in order to obtain the expression of a light chain and the expression of a heavy chain separately, a self-cleaving peptide (such as P2A) or a furin cleavage site is inserted between the light chain and the heavy chain, or the expression of the light chain and the expression of the heavy chain are driven by two promoters, respectively. However, we found that the design of this single vector may result in the low-level expression of antibodies in cells and the production of a large number of mispairing products. Low-level expression means that high doses of rAAV need to be administered in practical use to obtain effective antibody concentrations, and mispairing products are prone to form undesirable aggregates. In the field of drugs, these two issues are related to safety and toxicity and have received great concern.

The present invention provides a strategy for efficiently expressing antibodies in vivo by gene delivery mediated by dual rAAV vectors, wherein one rAAV vector contains a complete expression cassette containing a sequence encoding an antibody light chain, and the other rAAV vector contains a complete expression cassette containing a sequence encoding an antibody heavy chain; and when a cell is transduced simultaneously by using both vectors, the light and heavy chains expressed in the transduced cell may be paired and assembled to form a full-length functional antibody showing an enhanced antibody expression level. By adjusting a ratio of a light chain vector (LC vector) to a heavy chain vector (HC vector), the expression level of an antibody can be further improved, achieving more efficient antibody assembly and having a higher accuracy of pairing of heavy and light chains. When antibodies, in particular to bispecific antibodies or even multispecific antibodies, are expressed by using the dual rAAV vector system of the present invention, the antibodies can be expressed efficiently, and the system can be stably present in host cells for continuously expressing the antibodies, and has the excellent effects of high cell transformation efficiency, high antibody expression level and/or high accuracy of assembly of antibody heavy and light chains. The dual rAAV vector system of the present invention is particularly suitable for delivering an antibody to a subject so as to achieve the purpose of gene therapy, can effectively express an antibody in the body (such as in the body of a subject, including in the cell, tissue or organ of the subject), can be stably present in the body for continuously expressing the antibody, and does not need the integration of a sequence encoding an antibody into a genome of a cell in a subject, thereby having a high safety. The dual rAAV vector system of the present invention is particularly suitable for delivering an antibody to a non-dividing phase cell, for example, delivering an antibody to liver, eye, muscle and cerebrospinal fluid, such that the functional antibody is efficiently expressed in the cells of these tissues or body fluids.

Similarly, recombinant viral vectors, such as recombinant adenoviral vectors, recombinant herpes simplex viral vectors, recombinant lentiviral vectors and recombinant retroviral vectors, are used. Two recombinant viral vectors are used for delivering two chains of an antibody component separately, which also has the excellent effects of high cell transformation efficiency, high antibody expression level and/or high accuracy of assembly of antibody heavy and light chains.

Unless stated otherwise, the terms used in the present invention have the meanings commonly understood in the art and may be understood by reference to standard textbooks known to a person skilled in the art, reference books and literatures. The definitions and explanations provided herein serve to clarify the specific uses of the terms within the scope of the present invention. All references cited in the present invention are incorporated herein by reference in their entirety.

Without wishing to be bound by any particular theory, the explanations of the relevant theories or mechanisms in the present invention are intended to merely aid in the understanding of the present invention and should not be taken as limitation on the solutions of the present invention.

In the present invention, the term "contain/contains/containing", or other term forms having a meaning similar thereto such as "comprise/comprises/comprising", "include/includes/including" or "have/has/having", should be construed as including elements that are listed, but not excluding the presence of other elements; and these terms also include cases consisting only of the listed elements. The term "consists of/consisting of" refers to consisting only of the listed elements. The term "consist substantially of" refers to not excluding elements that do not significantly affect the solution involved.

Unless specifically stated otherwise, "a", "an" or "the" herein includes both singular and plural forms. The meaning of the term "at least one" or other similar expressions is equivalent to the meaning of "one or more".

The numerical value ranges described herein, such as temperature ranges, time ranges, composition or concentration ranges, or other numerical value ranges, include the end values within the ranges, all intermediate ranges and subranges (for example, a range between certain intermediate value and certain end value), and all individual numerical values, particularly intermediate ranges and subranges with integer values as end values, and individual integer values. In addition, any intermediate ranges, subranges and all individual numerical values described in the numerical value ranges can be excluded from the numerical value ranges.

The "about" and "approximately" described herein mean a range of ±20%, ±18%, ±15%, ±12%, ±10%, ±9%, ±8%, ±7%, ±6%, ±5%, ±4%, ±3%, ±2%, ±1% or ±0.5% of the value, wherein the range includes the endpoints of the range and any of the numerical values within the range.

The term "and/or" described herein should be construed as any one or a combination of the elements connected by the term.

In the present invention, the terms "nucleic acid sequence" and "nucleotide sequence" are used interchangeably and refer to a sequence formed by linking bases, sugars and a phosphate backbone. The nucleic acid sequence of the present invention can be a deoxyribonucleic acid sequence (DNA) or a ribonucleic acid sequence (RNA), and can comprise natural bases or unnatural bases, which can be single-stranded or double-stranded, and can be a coding sequence or a non-coding sequence.

In the present invention, unless otherwise stated, a nucleic acid sequence is generally described from a 5' end to a 3' end, and an amino acid sequence is generally described from N-terminus to C-terminus.

In the present invention, two recombinant viral vectors are used to respectively express two different peptide chains constituting a polypeptide molecule, the polypeptide molecule is composed of the two different peptide chains and has an antigen binding activity, and the two different peptide chains can be assembled, after being expressed in a cell, into a polypeptide molecule having antigen binding activity. Therefore, the present invention provides a dual recombinant viral vector system for expressing the polypeptide molecule, a dual plasmid system for producing the dual recombinant viral vector system, a host cell containing the dual recombinant viral vector system, a pharmaceutical composition, and the use of and the method for delivering the polypeptide molecule by using the dual recombinant viral vector system.

### Polypeptide molecule

The dual recombinant viral vector system of the present invention is suitable for expressing a polypeptide molecule which is composed of two different peptide chains and has an antigen binding activity.

The term "antigen binding activity" refers to an ability of the polypeptide molecule to specifically bind to and immunoreact with an antigenic epitope via an antigen binding site (such as a heavy chain variable region VH and/or a light chain variable region VL) contained in the polypeptide molecule. The "polypeptide molecule which is composed of two different peptide chains and has an antigen binding activity" of the present invention can be an antibody or an antibody fusion protein containing an antibody and a second binding domain. Two different peptide chains constituting the polypeptide molecule have different amino acid sequences and are referred to in the present invention as the first chain and the second chain respectively. The polypeptide molecule being composed of two different peptide chains refers to the polypeptide molecule being composed of one or more first chains and one or more second chains, for example, the polypeptide molecule can be composed of one first chain and one second chain, can also be a tetramer composed of two first chains and two second chains, wherein the first chain is expressed by one recombinant viral vector, the second chain is expressed by another recombinant vector, and the first and second chains can be assembled, after being expressed in a cell, into a polypeptide molecule having an antigen binding activity. The "assemble" comprises an interaction of a first chain and a second chain to form a polypeptide molecule having an antigen binding activity, wherein the interaction comprises interconnection via a covalent bond (such as a disulphide bond), for example, linking CH1, CH2 and/or CH3 of two heavy chain constant regions via a disulphide bond; and/or an interaction via a non-covalent bond (such as a hydrogen bond, van der Waals forces, a charge interaction or a hydrophobic interaction), for example, pairing between a heavy chain variable region and a light chain variable region.

In the present invention, using the expressions "first chain" and "second chain" is only intended to distinguish two different peptide chains constituting the polypeptide molecule, and does not mean that the first chain or the second chain contains a particular amino acid sequence or has a particular meaning. In the present invention, when describing specific embodiments, for the purpose of clarity, the first chain is described as containing a first sequence and the second chain is described as containing a second sequence, but a person skilled in the art should understand that the two can be used interchangeably, that is, the first chain can contain a second sequence and the second chain can contain a first sequence.

In the present invention, the term "antibody" can also be referred to as "antigen binding moiety", which refers to a polypeptide that contains an antigen binding site and can specifically bind to and immunoreact with an antigenic epitope. The term "antibody" comprises a polypeptide having a heavy chain variable region (VH) and a light chain variable region (VL), and also comprises a polypeptide having only a heavy chain variable region VH, such as a heavy chain antibody or a single domain antibody (sdAb). The term "antibody" comprises, but is not limited to, a monoclonal antibody or an antigen binding fragment thereof (such as Fab, Fab' and F(ab')₂ fragments), and an antibody in the form of a single chain (such as scFv or a single domain antibody sdAb), and also comprises a chimeric antibody, a humanized antibody, etc. The term "antibody" comprises a monospecific antibody, i.e., an antibody that specifically binds to a single antigenic epitope, and a multispecific antibody, i.e., an antibody that specifically binds to multiple different antigenic epitopes (such as a bispecific antibody and a trispecific antibody). In some embodiments, the "antibody" of the present invention is composed of two different chains. The two chains are linked to each other to assemble into an antibody having an antigen binding activity. The term "antibody in the form of a single chain" refers to an antibody composed of one chain. One chain contained in the antibody in the form of a single chain can contain at least a heavy chain variable region and a light chain variable region from immunoglobulin G (IgG), such as scFv, and can also contain at least a heavy chain variable region, such as a single domain antibody, or a heavy chain antibody (i.e., containing a heavy chain variable region and a heavy chain constant region). In the present invention, the term "specifically bind to/specifically binds to/specifically binding to" or "target/targets/targeting" refers to a selective recognition of an antigen binding polypeptide to a specific epitope of an antigen, that is, compared with different antigens or other epitopes of the same antigen, an antibody or an antigen binding moiety binds to the antigen or the specific epitope with a high affinity or for a long duration. The binding ability of an antibody to an antigenic epitope can be obtained, for example, by measuring a dissociation constant with radioimmunoassay (RIA).

The term "antigen" has the meaning commonly understood by those of ordinary skill in the art, and comprises polypeptides, polysaccharides, glycoproteins, polynucleotides, etc. The term "antigenic epitope" can also be referred to as "antigenic determinant", and refers to a chemical functional group on the surface of an antigen that determines the specificity of the antigen. The antigenic epitope can be recognized and bound by the antigen binding site in an antibody.

The term "monoclonal antibody" refers to a homogeneous antibody that targets only a specific antigenic epitope. Compared with common polyclonal antibody preparations which typically comprise different antibodies targeting different antigenic determinants (epitopes), each monoclonal antibody targets a single antigenic determinant on an antigen. Although monoclonal antibodies can be obtained by hybridoma culture, in the present invention, the modifier "monoclonal" indicates the homogeneous property of an antibody and should not be construed as an antibody that need to be produced by any specific method. The monoclonal antibody of the present invention can be produced by a DNA recombination method or obtained by other screening methods.

The "monoclonal antibody" of the present invention can also be referred to as a full-length antibody or a complete antibody, i.e., an immunoglobulin molecule composed of four polypeptide chains (such as IgG) or a polymer thereof (such as IgA or IgM). The four polypeptide chains comprise two identical heavy chains (H) and two identical light chains (L), which are linked to each other via disulphide bonds to form a tetramer. Each heavy chain is composed of a heavy chain variable region ("HCVR" or "VH") and a heavy chain constant region (composed of domains CH1, CH2 and CH3). Each light chain is composed of a light chain variable region ("LCVR" or "VL") and a light chain constant region (CL). VH and VL regions can be further subdivided into hypervariable regions called complementary determining regions (CDRs), which are interspersed with more conserved regions called framework regions (FRs). VH and VL each are composed of three CDRs and four FRs, which are arranged from an amino terminus to a carboxyl terminus in the following order: FR1, CDR1, FR2, CDR2, FR3, CDR3, and FR4. According to the amino acid sequence of a heavy chain constant region, immunoglobulin molecules can be classified into different categories, with five main classes of full-length antibodies: IgA, IgD, IgE, IgG and IgM. Some of these can be further divided into subclasses, such as IgG1, IgG2, IgG3, IgG4, IgA1 and IgA2. The monoclonal antibody of the present invention comprises immunoglobulin molecules of any of the foregoing classes or subclasses.

The "antigen binding fragment" of the present invention refers to one or more moieties of a complete antibody, wherein the moiety retains an ability to specifically interact with an antigenic epitope. Examples of the antigen binding fragment include: (1) Fab fragment: an antigen binding fragment produced by digesting a complete antibody with papain, consisting of a complete light chain (comprising a VL domain and a CL domain) of a full-length antibody, and a heavy chain variable region VH and a heavy chain constant region domain CH1 of the full-length antibody, without a hinge region; (2) F(ab')₂ fragment: an antigen binding fragment produced by digesting a complete antibody with pepsin, containing complete light chains (comprising a VL domain and a CL domain) of two full-length antibodies, and heavy chain variable regions VH, heavy chain constant region domains CH1 and hinge regions of the two full-length antibodies, which can be regarded as a fragment formed by pairing two Fab' fragments having a disulphide bond therebetween; (3) Fab' fragment; (4) Fd fragment composed of a VH domain and a CH1 domain; (5) Fv fragment composed of a VL domain and a VH domain of the single arm of an antibody; and (6) a single domain antibody sdAb composed of a VHH domain. In addition, the term "antigen binding fragment" of the present invention also comprises an antibody in the form of a single chain, such as scFv (single chain Fv formed by linking a VL domain and a VH domain via a linker) and dsFv (a single chain antibody formed by introducing an interchain disulphide bond between the VH and VL of scFv). The "antigen binding fragment" generally contains at least one or both of a heavy chain variable region and a light chain variable region of a complete antibody, and can also contain a portion of a light chain constant region and/or a heavy chain constant region of the complete antibody, for example, can contain one or two or more of a CL domain, a CH1 domain, a CH2 domain and a CH3 domain.

The term "single domain antibody (sdAb)" of the present invention refers to a single antigen binding polypeptide having three complementarity determining regions (CDRs). These single domain antibodies alone can bind to antigens without pairing with corresponding CDR-containing polypeptides. Common single domain antibodies contain a heavy chain but lacks common light chains in antibodies, such as camelid sdAb (see *e.g.,* Hamers-Casterman et al., Nature 363:446-8 (1993); Greenberg et al., Nature 374:168-73 (1995); Hassanzadeh-Ghassabeh et al., Nanomedicine (Lond), 8:1013-26 (2013)). The single domain antibody can be artificially engineered from a camel heavy chain antibody, called VHH, which has the following structure from the N-terminus to the C-terminus: FR1-CDR1-FR2-CDR2-FR3 -CDR3 -FR4.

The "single chain antibody (scFv)" of the present invention refers to a fusion protein formed by linking a heavy chain variable region and light chain variable region of an immunoglobulin via a peptide linker. The N-terminus of a heavy chain variable region can be linked to the C-terminus of a light chain variable region via a peptide linker, or the N-terminus of a light chain variable region can be linked to the C-terminus of a heavy chain variable region via a peptide linker.

The "multispecific antibody" of the present invention refers to an antibody capable of specifically binding to multiple antigenic epitopes. The multiple antigenic epitopes can be different epitopes on the same antigen, or can also be different epitopes on different antigens. The multispecific antibody contains multiple antigen binding moieties, each antigen binding moiety targets one antigenic epitope, and the antigenic epitopes targeted by the multiple antigen binding moieties are different from each other. Each antigen binding moiety can be independently selected from a monoclonal antibody (such as immunoglobulin G, IgG), an Fab fragment, an Fab' fragment, an F(ab')₂ fragment, an Fv fragment, an Fd fragment, scFv, dsFv, a single domain antibody, etc. According to different structures of multispecific antibodies, the first chain and second chain of the multispecific antibody can respectively contain a heavy chain variable region and/or a light chain variable region targeting one or two or more different antigenic epitopes.

In some preferred embodiments, the multispecific antibody is a bispecific antibody containing a first antigen binding moiety that specifically binds to a first antigenic epitope and a second antigen binding moiety that specifically binds to a second antigenic epitope, and the first antigenic epitope and the second antigenic epitope are different. The bispecific antibody has a variety of forms, and may or may not contain an Fc region, and in the bispecific antibody containing an Fc region, the structure thereof can be symmetrical or asymmetrical. In some preferred embodiments, the bispecific antibody of the present invention contains an Fc region. In some preferred embodiments, the bispecific antibody of the present invention is symmetric, and is composed of two identical first chains and two identical second chains. In some embodiments, the bispecific antibody of the present invention can also be composed of one first chain and one second chain. According to different structures of bispecific antibodies, the first chain and second chain of a bispecific antibody can respectively contain a heavy chain variable region and/or a light chain variable region targeting one antigenic epitope or two different antigenic epitopes. In some embodiments, the bispecific antibody of the present invention is symmetric, the first chain of the bispecific antibody contains a heavy chain targeting a first antigenic epitope, and the second chain of the bispecific antibody contains a light chain targeting a second antigenic epitope. In some embodiments, the bispecific antibody can be a bispecific antibody against VEGF and Ang2.

The "antibody fusion protein" of the present invention refers to a fusion protein having multiple biological functions obtained by fusing an antibody (which can be referred to as a first antigen binding moiety in the antibody fusion protein) with other polypeptides having biological functions (a second binding domain). The "other polypeptides having biological functions" of the present invention can be linked to a heavy chain variable region (VH), a light chain variable region (VL), heavy chain constant regions (CH1, CH2 and CH3), and/or a light chain constant region (CL) of the antibody. The "other polypeptides having biological functions" of the present invention can be another antibody, toxin, enzyme, cytokine, receptor or a fragment thereof, etc. targeting a different antigenic epitope. When the antibody is fused with another antibody targeting a different antigenic epitope, the antibody fusion protein is a multispecific antibody (such as a bispecific antibody). In some embodiments, the second binding domain can be a receptor that specifically binds to a ligand of the second binding domain, a fragment thereof (such as an extracellular domain thereof), or their variants. In some embodiments, the ligand of the receptor is a protein different from the antigen targeted by the first antigen binding moiety. In some embodiments, the ligand is a cytokine or a variant thereof, which cytokine can be, for example, a transforming growth factor β (TGFβ), a vascular endothelial growth factor (VEGF) receptor or angiotensin 2 (Ang2). In some embodiments, the second binding domain can be a receptor that specifically binds to these cytokines or variants thereof, such as a transforming growth factor β (TGFβ) receptor (such as TGF-βRII), a vascular endothelial growth factor (VEGF) receptor or an angiotensin 2 (Ang2) receptor, and can also be a fragment of the receptor, for example, a fragment capable of binding to a cytokine, for example, an extracellular domain of the receptor or a variant thereof. The cytokines and cytokine receptors can be from human. In some embodiments, the "other polypeptides" are an extracellular domain of TGFβRII or a variant thereof, such as an extracellular domain of TGFβRII (see, for example, SEQ ID NO: 13 of patent application WO 2018/205985), a soluble extracellular domain of TGFβRII (see, for example, SEQ ID NO: 10 of patent application WO 2018/129331), and an N-terminal truncated form of the extracellular domain of TGF-βRII (see, for example, SEQ ID NO: 14 or SEQ ID NO: 15 of patent application WO 2018/205985). The antibody fusion protein formed by fusing an antibody with a cytokine receptor or a fragment thereof can block an interaction between the cytokine receptor and a cytokine while the antibody plays a therapeutic role (for example, an anti-tumour effect), thereby achieving a synergistic therapeutic effect. For example, a receptor of TGFβ, such as TGFβRII or an extracellular domain thereof, can block the neutralization of TGFβ in a tumour microenvironment and can achieve a synergistic anti-tumour effect with an antibody; and a receptor of VEGF or Ang2 or an extracellular domain thereof can block the binding of VEGF or Ang2 to a receptor in the environment, thereby blocking angiogenesis and achieving a synergistic anti-tumour effect with an antibody. In preferred embodiments, the first antigen binding moiety is an anti-PD-1 antibody, an anti-PD-L1 antibody, an anti-VEGF antibody or an anti-Ang2 antibody.

In some embodiments, the polypeptide molecule of the present invention contains an antibody or an antigen binding moiety having a heavy chain variable region and a light chain variable region, and the heavy chain variable region and light chain variable region of the antibody or the antigen binding moiety are located on two peptide chains, respectively. In this case, the first chain and second chain of the polypeptide molecule respectively contain the heavy chain variable region and light chain variable region of the antibody or the antigen binding moiety, for example, the first chain of the polypeptide molecule contains the heavy chain variable region of the antibody or the antigen binding moiety, and the second chain contains the light chain variable region of the antibody or the antigen binding moiety. The dual recombinant viral vector system of the present invention can promote the correct pairing of the heavy chain variable region and the light chain variable region to form an active antigen binding moiety. Examples of such antibodies or antigen binding moieties include monoclonal antibodies or Fab fragments, Fab' fragments, (Fab')₂ fragments or Fc fragments thereof, etc. Further, other polypeptides having biological functions that are further fused in a first chain containing the heavy chain variable region and/or a second chain containing the light chain variable region are expressed by using the dual recombinant viral vector system of the present invention, which can still promote the correct pairing of the heavy chain variable region and the light chain variable region to form an active antigen binding moiety while producing other polypeptides having a biological activity. Therefore, the polypeptide molecule expressed by using the dual recombinant viral vector system of the present invention can be an antibody or an antigen binding moiety having a heavy chain variable region and a light chain variable region respectively located on two peptide chains, or a multispecific antibody composed of such antibody or antigen binding moiety and other antibodies or antigen binding moieties, or an antibody fusion protein composed of such antibody or antigen binding moiety and other proteins having biological functions.

Further, the first chain of the polypeptide molecule can further contain, in addition to the heavy chain variable region of the antibody or the antigen binding moiety, a full length or a portion of the heavy chain constant region CH, such as any one, any two or all of a CH1 domain, a CH2 domain and a CH3 domain, and the second chain can further contain, in addition to the light chain variable region of the antibody or the antigen binding moiety, a light chain constant region CL, such that an active antigen binding moiety is formed from the heavy chain variable region, the heavy chain constant region, the light chain variable region and the light chain constant region after the first chain and the second chain are expressed in a cell. Therefore, in preferred embodiments, the polypeptide molecule of the present invention contains an antibody or an antigen binding moiety having a heavy chain and a light chain. In this case, the first chain and second chain of the polypeptide molecule respectively contain the heavy chain and light chain of the antibody or the antigen binding moiety. For example, the first chain of the polypeptide molecule contains a heavy chain of the antibody or the antigen binding moiety, and the second chain contains a light chain of the antibody or the antigen binding moiety. Examples of such antibodies or antigen binding moieties include monoclonal antibodies or antigen binding fragments thereof containing a heavy chain and a light chain, such as Fab fragments, Fab' fragments and (Fab')₂ fragments. The inventors have found that using a dual recombinant viral vector system to respectively express the heavy chain and light chain of an antibody can make the expression of the antibody higher and the assembly efficiency of the heavy chain and light chain higher, and when the expressed light chain accounts for a higher proportion, the expression of the antibody and the assembly efficiency of the heavy chain and light chain are also higher.

In some embodiments, the antibody or the antigen binding moiety contained in the polypeptide molecule of the present invention is an antibody that specifically binds to VEGF or an antibody that specifically binds to Ang2. In some embodiments, the antibody that specifically binds to VEGF is Avastin. In some embodiments, the heavy chain of the antibody that specifically binds to VEGF has an amino acid sequence encoded by a nucleotide sequence as shown in SEQ ID NO: 2. In some embodiments, the light chain of the antibody that specifically binds to VEGF has an amino acid sequence encoded by a nucleotide sequence as shown in SEQ ID NO: 5. In some embodiments, the antibody that specifically binds to Ang2 is ABTAA. The heavy chain of the antibody that specifically binds to ABTAA has an amino acid sequence encoded by a nucleotide sequence as shown in SEQ ID NO: 7. In some embodiments, the light chain of the antibody that specifically binds to ABTAA has an amino acid sequence encoded by a nucleotide sequence as shown in SEQ ID NO: 10.

In the present invention, the term "heavy chain" refers to a chain containing a heavy chain variable region in a full-length antibody or an antigen binding fragment thereof containing at least two chains, and the term "light chain" refers to a chain containing a light chain variable region in a full-length antibody or an antigen binding fragment thereof containing at least two chains. Therefore, the "heavy chain" of the present invention refers to a chain containing a heavy chain variable region and at least part of heavy chain constant regions, such as a chain containing a heavy chain variable region VH and all three heavy chain constant regions CH1, CH2 and CH3, or a chain containing a heavy chain variable region VH and part of heavy chain constant regions (such as any one or any two of CH1, CH2 and CH3). The "light chain" of the present invention refers to a chain containing a light chain variable region and at least part of light chain constant regions, for example, a chain containing a light chain variable region VL and a light chain constant region CL.

Further, other polypeptides having biological functions (i.e., second binding domain) are further fused in a first chain containing the heavy chain and/or a second chain containing the light chain, which can still provide a high expression and a high assembly efficiency of heavy chain and light chain while produce other polypeptides having a biological activity. Therefore, the polypeptide molecule expressed by using the dual recombinant viral vector system of the present invention can be composed of an antibody or an antigen binding moiety having a heavy chain and a light chain, or a multispecific antibody composed of such antibody or antigen binding moiety and other antibodies or antigen binding moieties, or an antibody fusion protein composed of such antibody or antigen binding moiety and other proteins having biological functions.

In some preferred embodiments, the ratio of the first chain containing a heavy chain of an antibody to the second chain containing a light chain of the antibody can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4, wherein the first chain and the second chain are expressed by using the dual recombinant viral vector system of the present invention. The ratio of the first chain to the second chain can be determined according to the quantity of the viral genomes, containing sequences encoding the first chain and the second chain, of recombinant viral vectors, and the copy number of the sequences encoding the first chain and the second chain contained in the first recombinant viral vector and the second recombinant viral vector. In some embodiments, the sequence encoding the first chain in the first recombinant viral vector system is a single copy. In some embodiments, the sequence encoding the second chain in the second recombinant viral vector system is a single copy. In some embodiments, the sequences encoding functional protein domains (such as a heavy chain variable region, light chain variable region, heavy chain and light chain of an antibody and/or other polypeptides having biological functions) contained in the sequences encoding the first chain and the second chain are also single copies. In some embodiments, the ratio of the first chain to the second chain can be determined by the ratio of the first recombinant viral vector to the second recombinant viral vector. In some embodiments, the ratio of the first recombinant viral vector for expressing the first chain containing the heavy chain of an antibody to the second recombinant viral vector for expressing the second chain containing the light chain of the antibody can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4. The proportion of a recombinant viral vector can be calculated by the quantity of viral genomes, for example, according to viral genome titres. The determination of the quantity of viral genomes or the viral genome titres is well known to a person skilled in the art, for example, by PCR, such as droplet digital PCR (ddPCR).

In some preferred embodiments, the polypeptide molecule expressed by using the dual recombinant viral vector system of the present invention is a monoclonal antibody or an antigen binding fragment thereof containing a heavy chain and a light chain, such as an Fab fragment, an Fab' fragment and a (Fab')₂ fragment. The first chain contains a heavy chain of the monoclonal antibody or the antigen binding fragment, and the second chain contains a light chain of the monoclonal antibody or the antigen binding fragment. More preferably, the ratio of the first chain to the second chain can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4. In some embodiments, the ratio of the first recombinant viral vector for expressing the first chain to the second recombinant viral vector for expressing the second chain can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4.

In some other preferred embodiments, the polypeptide molecule expressed by using the dual recombinant viral vector system of the present invention is a bispecific antibody containing a first antigen binding moiety that specifically binds to a first antigenic epitope and a second antigen binding moiety that specifically binds to a second antigenic epitope, and the first antigenic epitope and the second antigenic epitope are different, wherein the first antigen binding moiety contains the heavy chain and light chain of an antibody, for example, a full-length antibody (such as immunoglobulin G, IgG) or an antigen binding fragment thereof containing a heavy chain and a light chain, such as an Fab fragment, an Fab' fragment and a (Fab')₂ fragment. The first chain contains the heavy chain of the first antigen binding moiety, and the second chain contains the light chain of the first antigen binding moiety. The bispecific antibody forms having different structures formed on this basis can be used in the present invention, for example, the second antigen binding moiety can be any single chain form or double chain form, for example, including but not limited to an Fab fragment, an Fv fragment, a scFv, a single domain antibody, etc. that specifically binds to a second antigenic epitope. When the second antigen binding moiety is an antibody in the form of a single chain, the second antigen binding moiety can be linked to the N-terminus of the heavy chain, the C-terminus of the heavy chain, the N-terminus of the light chain or the C-terminus of the light chain of the first antigen binding moiety, or can also be linked between the heavy chain variable region VH and heavy chain constant region CH1 of the first antigen binding moiety. When the second antigen binding moiety is an antibody in the form of a double chain, the two chains of the second antigen binding moiety can be respectively linked to the N-terminus or C-terminus of the two chains of the first antigen binding moiety. When the second antigen binding moiety is Fab, the "light chain variable region-light chain constant region (VL-CL)" in the second antigen binding moiety can be linked to the N-terminus of the heavy chain of the first antigen binding moiety, and the "heavy chain variable region-heavy chain constant region CH1 (VH-CH1)" in the second antigen binding moiety can be linked to the N-terminus of the light chain of the first antigen binding moiety. When the second antigen binding moiety is an Fv fragment, the heavy chain variable region of the second antigen binding moiety can be linked to the N-terminus of the heavy chain of the first antigen binding moiety, and the light chain variable region of the second antigen binding moiety can be linked to the N-terminus of the light chain of the first antigen binding moiety.

In some embodiments, the bispecific antibody is composed of an Fab fragment that specifically binds to a first antigenic epitope and two identical scFvs that specifically bind to a second antigenic epitope, and the two scFvs are linked to the N-terminus of the heavy chain and the N-terminus of the light chain of the Fab fragment, respectively. In this embodiment, the first chain contains the heavy chain of the Fab fragment that specifically binds to the first antigenic epitope and the scFv that specifically binds to the second antigenic epitope, and the second chain contains the light chain of the Fab fragment that specifically binds to the first antigenic epitope and the scFv that specifically binds to the second antigenic epitope.

In other embodiments, the bispecific antibody is composed of a monoclonal antibody against IgG that specifically binds to a first antigenic epitope and an Fab fragment that specifically binds to a second antigenic epitope, wherein the heavy chain of the Fab fragment is linked to the N-terminus of the light chain of the monoclonal antibody against IgG, and the light chain of the Fab fragment is linked to the N-terminus of the heavy chain of the monoclonal antibody against IgG. In this embodiment, the first chain contains the heavy chain of the monoclonal antibody against IgG that specifically binds to the first antigenic epitope and the light chain of the Fab fragment that specifically binds to the second antigenic epitope, and the second chain contains the light chain of the monoclonal antibody against IgG that specifically binds to the first antigenic epitope and the heavy chain of the Fab fragment that specifically binds to the second antigenic epitope.

In other embodiments, the bispecific antibody is composed of a monoclonal antibody against IgG that specifically binds to a first antigenic epitope and a single domain antibody that specifically binds to a second antigenic epitope, wherein the single domain antibody is linked to the N-terminus or C-terminus of the light chain of the monoclonal antibody against IgG, or to the N-terminus or C-terminus of the heavy chain of the monoclonal antibody against IgG, preferably, the single domain antibody is linked to the heavy chain of the monoclonal antibody against IgG (for example, the N-terminus or C-terminus of the heavy chain). In this embodiment, the first chain contains the heavy chain of the monoclonal antibody against IgG that specifically binds to the first antigenic epitope and the single domain antibody that specifically binds to the second antigenic epitope, and the second chain contains the light chain of the monoclonal antibody against IgG that specifically binds to the first antigenic epitope; or the first chain contains the heavy chain of the monoclonal antibody against IgG that specifically binds to the first antigenic epitope, and the second chain contains the light chain of the monoclonal antibody against IgG that specifically binds to the first antigenic epitope and the single domain antibody that specifically binds to the second antigenic epitope.

In other embodiments, the bispecific antibody is composed of a monoclonal antibody against IgG that specifically binds to a first antigenic epitope and an scFv that specifically binds to a second antigenic epitope, wherein the scFv is linked to the N-terminus or C-terminus of the light chain of the monoclonal antibody against IgG, or to the N-terminus or C-terminus of the heavy chain of the monoclonal antibody against IgG; preferably, the scFv is linked to the C-terminus of the heavy chain of the monoclonal antibody against IgG. In this embodiment, the first chain contains the heavy chain of the monoclonal antibody against IgG that specifically binds to the first antigenic epitope and the scFv that specifically binds to the second antigenic epitope, and the second chain contains the light chain of the monoclonal antibody against IgG that specifically binds to the first antigenic epitope; or the first chain contains the heavy chain of the monoclonal antibody against IgG that specifically binds to the first antigenic epitope, and the second chain contains the light chain of the monoclonal antibody against IgG that specifically binds to the first antigenic epitope and the scFv that specifically binds to the second antigenic epitope.

In other embodiments, the bispecific antibody is composed of an Fab fragment that specifically binds to a first antigenic epitope, an scFv that specifically binds to a second antigenic epitope and an Fc fragment, wherein the N-terminus of the scFv is linked to the C-terminus of the heavy chain of the Fab fragment, and the C-terminus of the scFv is linked to the N-terminus of the Fc fragment. The bispecific antibody of this type can also be regarded as being obtained by inserting scFv that specifically binds to a second antigenic epitope between the heavy chain constant regions CH1 and CH2 of a monoclonal antibody against IgG that specifically binds to a first antigenic epitope. In this embodiment, the first chain contains the heavy chain of the Fab fragment that specifically binds to the first antigenic epitope, the scFv that specifically binds to the second antigenic epitope, and the Fc fragment, and the second chain contains the light chain of the Fab fragment that specifically binds to the first antigenic epitope.

More preferably, in the dual recombinant viral vector system for expressing the bispecific antibody, the ratio of the first chain to the second chain can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4. In some embodiments, the ratio of the first recombinant viral vector for expressing the first chain to the second recombinant viral vector for expressing the second chain can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4.

In some other preferred embodiments, the polypeptide molecule expressed by using the dual recombinant viral vector system of the present invention can also be an antibody fusion protein containing a first antigen binding moiety that specifically binds to a first target molecule and a second binding domain that specifically binds to a second target molecule, wherein the first target molecule is an antigen or an antigenic epitope, the first antigen binding moiety is a full-length antibody (such as immunoglobulin G, IgG) or an antigen binding fragment thereof containing a heavy chain and a light chain, such as an Fab fragment, an Fab' fragment and a (Fab')₂ fragment. The first chain contains the heavy chain of the first antigen binding moiety, and the second chain contains the light chain of the first antigen binding moiety. The second target molecule is a cytokine or a variant thereof, and the second binding domain is a cytokine receptor, or an extracellular binding domain thereof, or a truncated form or variant thereof. The second target molecule is different from the first target molecule. The second binding domain can be linked to the heavy chain (for example, the N-terminus or C-terminus of the heavy chain) and/or the light chain (for example, the N-terminus or C-terminus of the light chain) of the first antigen binding moiety.

The term "variant" refers to a polynucleotide (or polypeptide) that has a sequence similarity to a parent nucleic acid (or polypeptide) and can retain the activity of the polynucleotide (or polypeptide). For example, the nucleic acid variant can have insertion, substitution and/or deletion of one or more nucleotides at a 5' end, a 3' end and/or one or more internal sites compared to a parent nucleic acid. The variant can also have at least approximately 70%, approximately 75%, approximately 80%, approximately 85%, approximately 90%, approximately 91%, approximately 92%, approximately 93%, approximately 94%, approximately 95%, approximately 96%, approximately 97%, approximately 98%, approximately 99% or more sequence identity to a parent nucleic acid. The variant of a polypeptide can have insertion, substitution and/or deletion of one or more amino acids at a 5' end, a 3' end and/or one or more internal sites compared to a parent polypeptide. The variant can also have at least approximately 70%, approximately 75%, approximately 80%, approximately 85%, approximately 90%, approximately 91%, approximately 92%, approximately 93%, approximately 94%, approximately 95%, approximately 96%, approximately 97%, approximately 98%, approximately 99% or more sequence identity to a parent polypeptide. Sequence identity can be determined by a sequence alignment program known to a person skilled in the art, including, but not limited to, BLAST, FASTA, Needleman, etc.

In some preferred embodiments, the second target molecule is a cytokine, such as a transforming growth factor β, a vascular endothelial growth factor (VEGF) or angiotensin (Ang2). In some embodiments, the second binding domain is a cytokine receptor, such as a transforming growth factor β (TGFβ) receptor (for example, TGF-βRII), a vascular endothelial growth factor (VEGF) receptor or an angiotensin (Ang2) receptor, or a fragment thereof (for example, an extracellular domain ECD), or their variants. The cytokines and cytokine receptors can be from human. In some embodiments, the second binding domain is an extracellular domain of TGFβRII or a variant thereof, such as an extracellular domain of TGFβRII (see, for example, SEQ ID NO: 13 of patent application WO 2018/205985), a soluble extracellular domain of TGFβRII (see, for example, SEQ ID NO: 10 of patent application WO 2018/129331), and an N-terminal truncated form of the extracellular domain of TGF-βRII (see, for example, SEQ ID NO: 14 or SEQ ID NO: 15 of patent application WO 2018/205985). In preferred embodiments, the first antigen binding moiety is an anti-PD-1 antibody, an anti-PD-L1 antibody, an anti-VEGF antibody or an anti-Ang2 antibody.

More preferably, in the dual recombinant viral vector system for expressing the antibody fusion protein, the ratio of the first chain to the second chain can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4. In some embodiments, the ratio of the first recombinant viral vector for expressing the first chain to the second recombinant viral vector for expressing the second chain can be 4 : 1-1 : 8, for example, the ratio can be 4 : 1-1 : 6, 2 : 1-1 : 6, 2 : 1-1 : 4, 1 : 1-1 : 4 or 1 : 2-1 : 4, for example, the ratio can be 1 : 1, 1 : 2, 1 : 3 or 1 : 4, most preferably 1 : 2-1 : 4.

The antibody or the antigen binding moiety (such as a monoclonal antibody or an antigen binding fragment thereof, a first antigen binding moiety and/or a second antigen binding moiety in a bispecific antibody, and a first antigen binding moiety in an antibody fusion protein) expressed by using the dual recombinant viral vector system of the present invention can be an antibody or an antigen binding moiety that specifically binds to any antigen including, but not limited to, a microbial antigen, a tumour-specific antigen, a cytokine, an angiogenesis-related target, an immune checkpoint, etc. In some particularly preferred embodiments, the antibody or the antigen binding moiety specifically binds to an angiogenesis-related target. The angiogenesis-related target refers to an angiogenic factor, i.e., a factor that stimulates vascular development, for example, promotes angiogenesis, endothelial cell growth and/or vascular stability. The angiogenic factor, for example, can be used as a target for treating angiogenesis-related diseases with inhibitors (for example, antibodies) against these angiogenic factors. The angiogenic factor includes, but is not limited to, a vascular endothelial growth factor (VEGF), a vascular endothelial growth factor receptor (VEGFR), a fibroblast growth factor (FGF), a platelet derived growth factor (PDGF), a transforming growth factor β (TGFβ), angiotensin 1 (Ang1), angiotensin 2 (Ang2), integrin, CD31 and a plasminogen activator. In some embodiments, the antibody or the antigen binding moiety of the present invention is an antibody against an angiogenic factor, and the angiogenic factor is one, two or more selected from a vascular endothelial growth factor (VEGF), a vascular endothelial growth factor receptor (VEGFR), a fibroblast growth factor (FGF), a platelet derived growth factor (PDGF), a transforming growth factor β (TGFβ), angiotensin 1 (Ang1), angiotensin 2 (Ang2), integrin, CD31 and a plasminogen activator. As is known to a person skilled in the art, when referring to specific binding of an antibody or an antigen binding moiety to a specific antigen, it is meant that the antibody or the antigen binding moiety specifically binds to any epitope on the antigen.

In some embodiments, the antibody or the antigen binding moiety can be an antibody against a vascular endothelial growth factor (VEGF) and/or an antibody against angiotensin 2 (Ang2). In some embodiments, the anti-VEGF antibody is Avastin. In some embodiments, the anti-VEGF antibody has a heavy chain CDR sequence that is identical to three heavy chain CDR sequences contained in a heavy chain sequence as shown in SEQ ID NO: 19 (for example, which can be encoded by a nucleotide sequence as shown in SEQ ID NO: 2). In some embodiments, the anti-VEGF antibody has a heavy chain variable region sequence that is identical to a heavy chain variable region sequence contained in a heavy chain sequence as shown in SEQ ID NO: 19 (for example, which can be encoded by a nucleotide sequence as shown in SEQ ID NO: 2). In some embodiments, the anti-VEGF antibody has a heavy chain sequence as shown in SEQ ID NO: 19 (for example, which can be encoded by a nucleotide sequence as shown in SEQ ID NO: 2). In some embodiments, the anti-VEGF antibody has a light chain CDR sequence that is identical to three light chain CDR sequences contained in a light chain sequence as shown in SEQ ID NO: 20 (for example, which can be encoded by a nucleotide sequence as shown in SEQ ID NO: 5). In some embodiments, the anti-VEGF antibody has a light chain variable region sequence that is identical to a light chain variable region sequence contained in a light chain sequence as shown in SEQ ID NO: 20 (for example, which can be encoded by a nucleotide sequence as shown in SEQ ID NO: 5). In some embodiments, the anti-VEGF antibody has a light chain sequence as shown in SEQ ID NO: 20 (for example, which can be encoded by a nucleotide sequence as shown in SEQ ID NO: 5). In some embodiments, the anti-Ang2 antibody is ABTAA. In some embodiments, the anti-Ang2 antibody has a heavy chain CDR sequence that is identical to three heavy chain CDR sequences contained in a heavy chain variable region sequence as shown in SEQ ID NO: 21, or has a heavy chain CDR sequence that is identical to three heavy chain CDR sequences contained in a heavy chain sequence encoded by a nucleotide sequence as shown in SEQ ID NO: 7. In some embodiments, the anti-Ang2 antibody has a heavy chain variable region sequence as shown in SEQ ID NO: 21, or has a heavy chain variable region sequence that is identical to a heavy chain variable region sequence contained in a heavy chain sequence encoded by a nucleotide sequence as shown in SEQ ID NO: 7. In some embodiments, the anti-Ang2 antibody has a light chain CDR sequence that is identical to three light chain CDR sequences contained in a light chain variable region sequence as shown in SEQ ID NO: 22, or has a light chain CDR sequence that is identical to three light chain CDR sequences contained in a light chain sequence encoded by a nucleotide sequence as shown in SEQ ID NO: 10. In some embodiments, the anti-Ang2 antibody has a light chain variable region sequence as shown in SEQ ID NO: 22, or has a light chain variable region sequence that is identical to a light chain variable region sequence contained in a light chain sequence encoded by a nucleotide sequence as shown in SEQ ID NO: 10. In some embodiments, the heavy chain of the anti-Ang2 antibody has an amino acid sequence encoded by a nucleotide sequence as shown in SEQ ID NO: 7. In some embodiments, the light chain of the anti-Ang2 antibody has an amino acid sequence encoded by a nucleotide sequence as shown in SEQ ID NO: 10.

It should be understood that, in the present invention, when referring to the linking of a certain amino acid sequence to the N-terminus of another amino acid sequence, it is meant that the C-terminus of the former is linked to the N-terminus of the latter, and when referring to the linking of a certain amino acid sequence to the C-terminus of another amino acid sequence, it is meant that the N-terminus of the former is linked to the C-terminus of the latter. In the present invention, when referring to the linking or connecting of two peptide chains or two amino acid sequences, it should be understood that the two peptide chains or the two amino acid sequences can be linked either directly or via a peptide linker (or referred to a linker peptide). The peptide linker can contain or be composed of glycine rich in flexibility and serine or threonine having solubility. The peptide linker can be, for example, a GS linker, which, for example, can be from 5 to approximately 25 amino acids in length. "GS linker" refers to a GS combination of glycine (G) and serine (S), which is used for linking multiple proteins together to form a fusion protein. A common GS combination is (GGGGS)n, and the length of a linker sequence can be changed by changing the size of n. For example, (GGGGS)₃ (i.e., GGGGSGGGGSGGGGS, SEQ ID NO: 16) can be used, and the linker, for example, can be encoded by a sequence as shown in SEQ ID NO: 11. Different linker sequences can also be produced by other combinations of glycine and serine, for example, GGGGSGGGS (SEQ ID NO: 17). The peptide linker can also be other sequences, for example, ESKYGPPSPPSP (SEQ ID NO: 23), which peptide linker, for example, can be encoded by a nucleic acid sequence as shown in SEQ ID NO: 14. Suitable peptide linkers can be determined by a person skilled in the art.

A person skilled in the art should understand that the first chain and/or the second chain can also contain a leader sequence (i.e., a signal peptide) that allows the secretion of the encoded polypeptide, such that the first chain and/or the second chain is produced in a secreted form from a cell transduced with a recombinant viral vector. The signal peptide can be placed at the N-terminus of the first chain and/or the second chain. In some embodiments, the polypeptide molecule is a monoclonal antibody or an antigen binding fragment thereof, wherein the first chain sequentially contains a leader sequence and the heavy chain of a monoclonal antibody or an antigen binding fragment thereof from the N-terminus to the C-terminus, and the second chain sequentially contains a leader sequence and the light chain of the monoclonal antibody or the antigen binding fragment thereof from the N-terminus to the C-terminus. In some embodiments, the polypeptide molecule is a bispecific antibody, wherein the first chain sequentially contains a leader sequence, the heavy chain of a first antigen binding moiety, a linker peptide and a second antigen binding moiety in the form of a single chain from the N-terminus to the C-terminus, and the second chain sequentially contains a leader sequence and the light chain of the first antigen binding moiety from the N-terminus to the C-terminus. In some embodiments, the polypeptide molecule is an antibody fusion protein, wherein the first chain sequentially contains a leader sequence, the heavy chain of a first antigen binding moiety, a linker peptide and a second binding domain from the N-terminus to the C-terminus, and the second chain sequentially contains a leader sequence and the light chain of the first antigen binding moiety from the N-terminus to the C-terminus. In some embodiments, the leader sequence can be MGWSCIILFLVATATGVHS (SEQ ID NO: 18). In some embodiments, the nucleotide sequence encoding the leader sequence is SEQ ID NO: 1, SEQ ID NO: 4, SEQ ID NO: 6 or SEQ ID NO: 9.

### Recombinant viral vector

The term "vector" of the present invention refers to any one of molecules, such as a nucleic acid, a plasmid or a virus, capable of transporting a heterologous nucleic acid contained therein into a host cell (the heterologous nucleic acid being present in a free form or being integrated into the genome of the host cell).

The term "recombinant" of the present invention refers to a vector containing a heterologous sequence which is not a native sequence of the vector, wherein the heterologous sequence can be a nucleic acid of interest for expressing a target protein, such as the polypeptide molecule of the present invention.

The "heterologous nucleic acid", "heterologous sequence" or "nucleic acid of interest" of the present invention can be used interchangeably, and refers to a nucleic acid sequence that is not natively present in a vector or host cell containing same. The heterologous nucleic acid can be a nucleic acid sequence encoding a target protein, for example, encoding the polypeptide molecule of the present invention.

The term "recombinant viral vector", "recombinant virus" or "recombinant viral particle" used in the present invention can be used interchangeably, and refer to a recombinant virus or a recombinant viral particle capable of mediating the transfer of a nucleic acid of interest (for example, nucleic acid sequences encoding first and second chains contained in the polypeptide molecule of the present invention) into a cell and allowing the expression of a polypeptide product, generally containing a recombinant viral genome packaged in the viral particle (for example, packaged in a viral capsid, some viruses also having envelope glycoproteins), wherein the recombinant viral genome contains the nucleic acid of interest. A recombinant viral vector capable of being used for expressing the polypeptide molecule of the present invention can be a viral vector derived from various types of viruses, including, but not limited to, an adeno-associated virus (AAV) vector, an adenoviral vector, a lentiviral vector, a retroviral vector, a herpes simplex virus (HSV) vector, etc.

Methods for producing recombinant viral vectors vary depending on the type of viral vectors used, but are well known to a person skilled in the art. Generally, an expression plasmid containing a recombinant viral genome (generally containing at least a heterologous nucleic acid and a cis-nucleic acid element, such as a terminal repeat sequence and a packaging signal sequence, required for virus production), a packaging plasmid and optionally other helper plasmids can be used to co-transfect a packaging cell line to obtain a recombinant viral vector. The co-transfected packaging cell line can be cultured under suitable conditions and viral particles are collected from the cell lysate and/or the culture supernatant. When a viral nucleic acid element contained in a packaging plasmid is already contained in a packaging cell line, the packaging cell line can be transfected without the packaging plasmid and only with an expression plasmid. Culture media for producing recombinant viral vectors are known to a person skilled in the art, and commercially available or customized culture media can be used, or one or more cell culture components known in the art, including but not limited to glucose, vitamins, amino acids, and/or growth factors, can further be added and supplemented to increase the titre of recombinant viral particles in a produced culture. The recombinant viral vector production culture can grow under conditions suitable for a particular host cell (i.e., packaging cell) used. Following the production of a recombinant viral vector, viral particles can be purified from a cell lysate or a viral supernatant, if required, by using a variety of conventional methods, such methods including column chromatography, CsCl gradient method, etc. For example, multiple column purification steps can be used, such as purification on an anion exchange column, an affinity column, and/or a cation exchange column.

The "transfection" of the present invention refers to the transfer of a polynucleotide, such as a nucleic acid molecule and a plasmid, from outside of a cell into the cell such that the polynucleotide is functional within the cell. Transfection methods are well known to a person skilled in the art, for example, transfection mediated by calcium phosphate or liposome.

Various types of cells can be used as packaging cell lines. For example, packaging cell lines that can be used include, but are not limited to, mammalian (including human) cells, insect cells, plant cells, microorganisms or yeasts, such as HEK293 cell lines (such as HEK293A, HEK293T or HEK293FT), HeLa cells, A549 cells, Vero cells, hARPE-19 cells, and insect-derived cell lines such as SF-9. As is known to a person skilled in the art, suitable packaging cell lines can be selected depending on the different types of recombinant viral vectors.

The first recombinant viral vector and second recombinant viral vector of the present invention can be viral vectors derived from the same type of virus or different types of viruses, for example, both can be adeno-associated virus (AAV) vectors, adenoviral vectors, herpes simplex virus (HSV) vectors, lentiviral vectors and retroviral vectors. In some embodiments, the first recombinant viral vector and the second recombinant viral vector contain the same viral elements (including nucleic acid elements, capsids, and optionally envelope glycoproteins, etc.), and can differ only in a heterologous nucleic acid contained. In some embodiments, the first recombinant viral vector and the second recombinant viral vector, although derived from the same type of virus, can contain the same or different viral elements, for example, the two can be derived from different serotypes of the same type of virus, respectively.

In the dual recombinant viral vector system of the present invention, the first recombinant viral vector and the second recombinant viral vector of the present invention can be provided in the form of a composition, for example, the two can be mixed with each other, or can be provided separately in separate forms, that is, the two are not mixed with each other.

The "recombinant viral vector genome (vg)" or "recombinant viral genome (vg)" of the present invention can be used interchangeably, and refers to a polynucleotide sequence that can be packaged in a viral capsid, which polynucleotide sequence contains at least an expression cassette containing a nucleic acid of interest and a cis-nucleic acid element required for viral production, and can also contain other viral nucleic acid elements. According to different types of recombinant viral vectors, the genome of a recombinant viral vector can contain single-stranded DNA, double-stranded DNA, or single-stranded RNA or double-stranded RNA. In some embodiments, the quantity or titre of the genome of a recombinant viral vector can be measured in vg/mL.

The term "expression cassette" refers to a segment of linear nucleic acid, containing a nucleic acid of interest operably linked to an expression regulatory sequence. The nucleic acid of interest encodes an expression product, for example, the first chain or second chain of the polypeptide molecule of the present invention. The expression regulatory sequence refers to a sequence that has a regulatory effect on a process of expressing a target polypeptide from a nucleic acid of interest, including but not limited to a promoter, an enhancer, a terminator, a selectable marker gene, a post-transcriptional regulatory element, an internal ribosome entry site (IRES) and/or a polyadenylation signal, etc. In the present invention, the first expression cassette for expressing the first chain and the second expression cassette for expressing the second chain can contain the same or different expression regulatory sequences, for example, the same or different promoters, the same or different enhancers, the same or different terminators, the same or different selectable marker genes, the same or different post-transcriptional regulatory elements, the same or different internal ribosome entry sites (IRES) and/or the same or different polyadenylation signals can be contained.

The promoters that can be used in the present invention include, but are not limited to, promoters from viruses, mammals (including human), bacteria, yeasts, plants, etc. The promoters can be constitutive, inducible, or cell- or tissue-specific. Examples of viral promoters include, but are not limited to, a cytomegalovirus (CMV) immediate early promoter, an RSV promoter, a chicken β-actin (CBA) promoter, a CMV early enhancer/chicken β-actin (CAG) promoter, a simian virus 40 (SV40) promoter, 35SRNA and 19SRNA promoters of cauliflower mosaic virus (CaMV), a coat protein promoter of tobacco mosaic virus (TMV), etc. Examples of plant promoters include, but are not limited to, a heat shock promoter. Examples of mammalian promoters include, but are not limited to, a human elongation factor 1a (EF1a) promoter, a human ubiquitin C (UCB) promoter, a mouse phosphoglycerate kinase (PGK) promoter, an RNA polymerase III promoter (such as U6 and H1), etc. Examples of constitutive promoters include, but are not limited to, a retrovirus, i.e., roussarcoma virus (RSV), LTR promoter, a cytomegalovirus (CMV) promoter, an SV40 promoter, a dihydrofolate reductase promoter, a β-actin promoter, a phosphoglycerate kinase (PGK) promoter and an EF1a promoter. Examples of inducible promoters include, but are not limited to, a zinc-inducible sheep metallothionein (MT) promoter, a dexamethasone (Dex)-inducible mouse mammary tumour virus (MMTV) promoter, a T7 polymerase promoter system, etc. Cell- or tissue-specific promoters can initiate the expression of a gene on a specific tissue or cell type and thus can be used to deliver the polypeptide molecule of the present invention to a specific cell, tissue or organ, examples of which include, but are not limited to, a rhodopsin kinase (RK) promoter capable of driving the expression of a nucleic acid of interest in rod and cone photoreceptor cells and retinal cell lines, a liver-specific TTR promoter, a liver-specific human α1-antitrypsin (hAAT) promoter, a liver-specific TBG promoter, a mature neuron-specific hSyn promoter, a projection neuron-specific CaMKIIa promoter, an astrocyte-specific GfaABC1D promoter, and a myocardial-specific cTNT promoter. When the dual recombinant viral vector system of the present invention is used to deliver a polypeptide molecule for gene therapy, it is expected that the polypeptide molecule is expressed in cells in a subject, and therefore promoters are preferably from viruses and mammalian (including human). When a host cell is used to produce the polypeptide molecule of the present invention, any source of promoter having a promoter activity in the host cell can be used.

Post-transcriptional regulatory elements can increase the expression level of a polypeptide of interest in a host cell. The post-transcriptional regulatory element that can be used in the present invention, for example, can be a virus post-transcriptional regulatory element such as a woodchuck hepatitis virus post-transcriptional regulatory element (WPRE), a hepatitis B virus post-transcriptional regulatory element (HBVPRE), an RNA transport element (RTE), etc.

The expression cassette can also comprise a gene for a selectable marker that is effective in a cell (such as a packaging cell line for producing a recombinant viral vector, or a cell transduced with a recombinant viral vector), for example, a drug resistance selectable marker. Such selectable marker gene can encode a factor necessary for the survival or growth of a cell growing in a selective medium. The host cell that is not transformed with a vector containing a selectable gene cannot survive in a culture medium. Proteins that confer resistance to antibiotics or other toxins, complement auxotrophic deficiencies or supply critical nutritional elements withheld from culture media can be selected as selectable markers, and examples of antibiotics or other toxins include but are not limited to ampicillin, hygromycin, neomycin, methotrexate, kanamycin, gentamycin, Zeocin, or tetracycline.

The polyadenylation signal sequence can be located in a transcription termination region, for example, a 3' untranslated region, examples of which include, but are not limited to, a bovine growth hormone (BGH) poly (A) sequence, an SV40 late poly (A) sequence, a thymidine kinase (TK) poly (A) sequence, etc.

The present invention also provides a dual plasmid system for producing the dual recombinant viral vector system, comprising: a first plasmid for producing a first recombinant viral vector and a second plasmid for producing a second recombinant viral vector, respectively. The first plasmid contains a recombinant viral genome containing a first expression cassette, and the second plasmid contains a recombinant viral genome containing a second expression cassette. The first plasmid and second plasmid are co-transfected with an optional packaging plasmid and optionally other helper plasmids, respectively, into a packaging cell line to obtain a first recombinant viral vector and a second recombinant viral vector. For different types of recombinant viral vectors, it is known to a person skilled in the art how to construct a plasmid containing a recombinant viral genome and used for producing a recombinant viral vector, a packaging plasmid and other helper plasmids, and it is also known to how to select a packaging cell line and how to co-transfect a packaging cell line with these plasmids to obtain a recombinant viral vector.

### Recombinant AAV vector (rAAV vector)

The recombinant viral vector of the present invention (such as a first recombinant viral vector and/or a second recombinant viral vector) can be a recombinant adeno-associated viral vector. Adeno-associated virus (AAV) is a non-enveloped and naturally replication-defective DNA virus, which belongs to a parvovirus, and has a single-stranded DNA genome of approximately 4.7 kb in length, including genes encoding a replication protein (Rep) and a virus capsid (Cap), and two 145 bp inverted terminal repeats (ITRs) flanking the genes. The ITRs are the minimal self-sequences necessary for AAV replication and packaging, and are also cis-acting elements necessary for AAV integration, replication and packaging. Therefore, the other portions of an AAV genome can be replaced with an expression cassette containing a nucleic acid of interest, while genes encoding Rep and Cap are provided in trans on another plasmid to produce a recombinant AAV vector. When an AAV vector does not contain rep and cap genes in the viral genome thereof, the AAV vector can be present in the nucleus of a transduced cell in the form of episome, rather than integrating into the genome of the transduced cell. In natural systems, infection with AAV requires the involvement of a helper virus (such as adenovirus or herpesvirus), and the helper virus provides a gene, such as E1A, E1B, E2A, E4 and VA, which allows AAV to replicate in an infected cell. In the production of a recombinant AAV vector, instead of using a helper virus, a plasmid containing an essential gene contained in a helper virus can be used for providing corresponding auxiliary function.

The "recombinant AAV vector" of the present invention refers to an AAV particle that contains a structural and/or functional nucleic acid element derived from AAV and is capable of transferring a nucleic acid of interest contained in the viral genome thereof into a cell, generally containing an AAV capsid and a recombinant viral genome packaged in the capsid, wherein the recombinant viral genome contains an expression cassette for expressing the first chain or the second chain of the polypeptide molecule of the present invention. As is known to a person skilled in the art, the viral genome also contains AAV inverted terminal repeats (ITRs) flanking the expression cassette.

AAV has different serotypes, including AAV1, AAV2, AAV3, AAV4, AAV5, AAV6, AAV7, AAV8, AAV9, AAV10, AAV11, AAV12, AAV13, AAV Rh10, etc. Capsid proteins of AAV of each serotype can bind to different receptors, for example, AAV2 binds to heparan sulphate proteoglycan (HSPG), AA1, AAV5 and AAV6 bind to N-linked sialic acid, AAV3 binds to heparan, and AAV9 binds to an N-terminal galactose residue, thereby rendering different targeting properties of AAV having different serotypes to different cells, tissues or organs. For example, AAVs 1-9 can target multiple types of retinal cells, such as pigment epithelium and photoreceptor cells; AAV8 has hepatocyte tropism in mice; AAV8 and AAV9 can target multiple muscle types throughout the body, and AAV9 is more tropism for muscle; and AAV9 can cross the blood-brain barrier (BBB) in mice. A person skilled in the art can understand the tropism of different AAV serotypes and AAV vectors having different AAV capsids for different cells, tissues and organs.

In the present invention, the first recombinant AAV vector and/or the second recombinant AAV vector can be constructed by using nucleic acid elements derived from AAVs having various different serotypes, particularly, the serotype of a capsid can be selected according to a cell, tissue and/or organ desired to be targeted. In particular, in the present invention, the first recombinant AAV vector and the second recombinant AAV vector can each independently use a capsid from AAV1, AAV2, AAV3, AAV4, AAV5, AAV6, AAV7, AAV8, AAV9, AAV10, AAV11, AAV12, AAV13 or AAV Rh10, a chimeric AAV capsid or a modified AAV capsid. In preferred embodiments, the first recombinant AAV vector and the second recombinant AAV vector contain capsids having the same tropism, for example, contain capsids capable of targeting the same cells, tissues and/or organs, preferably contain capsids from the same AAV serotype. In some embodiments, the first recombinant AAV vector and/or the second recombinant AAV vector contains a capsid from AAV2, AAV5, AAV8 or AAV9.

The chimeric AAV capsid or the modified AAV capsid can be used for changing the tropism of an AAV vector and changing or increasing the targeting property of an AAV vector to a specific cell, tissue and/or organ. The term "tropism" refers to the targeting property of a recombinant viral vector to a specific cell, tissue and/or organ. The term "chimeric AAV capsid" refers to an AAV capsid protein comprising amino acid sequences (for example, domains) from two or more different AAV serotypes and capable of forming an AAV capsid, examples of which include, but are not limited to, a capsid of an AAV DJ. The term "modified AAV capsid" refers to an AAV capsid protein comprising insertion, substitution or deletion of one or more amino acids, or an AAV capsid formed by the insertion of a polypeptide derived from other binding domains, examples of which include, but are not limited to, AAV2-7m8. In some embodiments, the first recombinant AAV vector and/or the second recombinant AAV vector contains ITRs from AAV1, AAV2, AAV3, AAV4, AAV5, AAV6, AAV7, AAV8, AAV9, AAV10, AAV11, AAV12, AAV13 or AAV Rh10. ITRs contained in the first recombinant AAV vector and the second recombinant AAV vector can be ITRs from the same AAV serotype or ITRs from different AAV serotypes. In some embodiments, the ITRs of the first recombinant AAV vector and/or the second recombinant AAV vector are from AAV2, AAV5, AAV8 or AAV9. In some embodiments, the ITRs of the first recombinant AAV vector and the second recombinant AAV vector are from the same AAV serotype, for example, all from AAV2, AAV5, AAV8 or AAV9.

In the first recombinant AAV vector and/or the second recombinant AAV vector of the present invention, the capsid and the ITRs can be derived from the same AAV serotype or different AAV serotypes. In some embodiments, in the first recombinant AAV vector and/or the second recombinant AAV vector, the ITRs and the capsid can be derived from different AAV serotypes, for example, ITRs of AAV2 can be selected for use with a capsid which is not from AAV2, such as a capsid of AAV5, AAV8 or AAV9. In some embodiments, in the first recombinant AAV vector and/or the second recombinant AAV vector, the ITRs and the capsid are from the same AAV serotype, for example, all from AAV2, AAV5, AAV8 or AAV9. In preferred embodiments, the ITRs of the first recombinant AAV vector and the second recombinant AAV vector are from the same serotype, such as AAV2; and the capsids of the first recombinant AAV vector and the second recombinant AAV vector are also from the same AAV serotype, such as AAV2, AAV5, AAV8 or AAV9.

Methods for producing a recombinant AAV vector are known to a person skilled in the art, for example, a packaging cell line can be provided with a nucleic acid molecule containing a recombinant AAV genome, a nucleic acid molecule containing nucleotides encoding AAV rep and cap protein, and a nucleic acid molecule containing a helper virus (such as adenovirus or herpes simplex virus) gene, thereby producing a recombinant AAV. The cap and rep genes of AAV can also be integrated into a packaging cell in advance such that the packaging cell line is transfected only with a nucleic acid molecule containing a recombinant AAV genome and a nucleic acid molecule containing a helper virus gene during production. The nucleic acid molecule, for example, can be provided by a plasmid. Recombinant AAV production methods that can be used include a three-plasmid co-transfection method, that is, a packaging cell line is co-transfected with a transfer plasmid containing a recombinant AAV genome, pRC plasmid containing the rep and cap genes of AAV, and pHelper plasmid containing helper genes (such as E2A, E4 and VA RNA genes), the cells are cultured under suitable conditions, and a recombinant AAV vector is then isolated and purified from the cell lysate and/or the culture supernatant, wherein the transfer plasmid for producing the recombinant AAV expression vector can contain an expression cassette and AAV inverted terminal repeats flanking the expression cassette.

### Recombinant adenoviral vector

The recombinant viral vector of the present invention (such as the first recombinant viral vector and/or the second recombinant viral vector) can be a recombinant adenoviral vector. Adenovirus is a non-enveloped linear double-stranded DNA virus with a genome of approximately 36 kb. The adenoviral genome contains inverted terminal repeats (ITRs) at both ends, a packaging signal sequence, early genes (genes E1, E2, E3 and E4 encoding regulatory proteins) and late genes (genes L1-L5 encoding structural proteins). Adenoviruses have more than 50 serotypes and are classified into 6 different subgroups: A-F. The recombinant viral vector of the present invention can be derived from any one of serotypes of adenovirus, preferably a subgroup C adenovirus or a variant thereof, such as Ad2, Ad5 and Ad55. E3 is a region that is non-essential for viral replication, and deletion of E3 region does not affect the production of a recombinant adenoviral vector. Deletion of E1A and/or E1B may result in a recombinant virus unable to replicate, thereby producing a replication-defective adenoviral vector. The adenoviral vector of the present invention can be a defective adenoviral vector, for example, a replication-defective adenoviral vector, with deletion of E1A and/or E1B. In the production of a recombinant adenoviral vector, late genes, E1, E2, E3 and/or E4 can be provided in trans by additional nucleic acid molecules (such as plasmids), so that all or a portion of these genes in a lentiviral genome can be deleted, and are replaced with expression cassettes containing nucleic acids of interest, while late genes, E1, E2, E3 and/or E4 are provided in trans on one or more additional nucleic acid molecules (such as plasmids) to produce a recombinant lentiviral vector. In some embodiments, E3 may not be provided. In some embodiments, the recombinant adenoviral vector is replication-defective, with the deletion of at least a portion of E1; at this time, E2 and E4 are provided in trans on additional nucleic acid molecules (such as plasmids), and E1 can be provided by a packaging cell line, such as a cell line derived from HEK293 and a PER.C6 cell line, which contain the E1 region of adenovirus.

The "recombinant adenoviral vector" of the present invention refers to an adenoviral particle that contains a structural and/or functional nucleic acid element derived from an adenovirus and is capable of transferring a nucleic acid of interest contained in the viral genome thereof into a cell, generally containing an adenoviral capsid and a recombinant adenoviral genome packaged in the capsid, wherein the recombinant adenoviral genome contains an expression cassette for expressing the first chain or second chain of the polypeptide molecule of the present invention. As is known to a person skilled in the art, a recombinant adenoviral genome also contains at least inverted terminal repeats (5' ITR and 3' ITR) at both ends of the genome and a sequence of packaging signal Ψ. The recombinant adenoviral vector of the present invention can be derived from Ad2, Ad5 and Ad55.

The recombinant adenoviral vector containing a nucleic acid of interest can be prepared by any technique known to a person skilled in the art, for example, by the AdEasy method or the AdMAX method known in the art. For example, a recombinant adenoviral vector can be obtained by transfecting a packaging cell line with a shuttle plasmid containing a nucleic acid of interest and an adenoviral backbone plasmid containing a portion of an adenoviral gene. The shuttle plasmid contains a recombinant adenoviral genome, which contains an expression cassette (in which a nucleic acid of interest, such as the first expression cassette or the second expression cassette of the present invention, is contained) and cis-nucleic acid elements, such as packaging signal sequences such as inverted terminal repeats, necessary for the production of a recombinant adenovirus. The adenoviral backbone plasmid can contain late genes, E1, E2, E3 and/or E4, and does not contain a packaging signal sequence. A shuttle plasmid and an adenoviral backbone plasmid can be transfected into a packaging cell line and allowed to undergo homologous recombination or site-specific recombination in a cell, thereby producing a recombinant adenovirus. The site-specific recombination, for example, can use a Cre/loxP recombinase system.

In the present invention, the first recombinant viral vector and the second recombinant viral vector can be derived from the same type of adenovirus or different types of adenoviruses, and when both are derived from the same type of adenovirus, they can contain the same or different viral elements, such as the same or different 5' ITRs, 3' ITRs, sequences of packaging signal Ψ, and late genes, E1, E2, E3 and/or E4.

### Recombinant retroviral vector

The recombinant viral vector of the present invention (such as the first recombinant viral vector and/or the second recombinant viral vector) can be a recombinant retroviral vector. Retrovirus is an RNA virus, and the viral genome is RNA. When a host cell is infected with a retrovirus, the viral genomic RNA is reverse-transcribed into a DNA intermediate and integrated into the chromosomal DNA of the infected cell. Retroviral genome and proviral DNA have three genes: gag, pol and env, which are flanked by two long terminal repeat (LTR) sequences, with a sequence of packaging signal Ψ between a 5' LTR and a gag gene. The gag gene encodes internal structure (matrix, capsid and nucleocapsid) proteins, and the env gene encodes a viral envelope glycoprotein. The pol gene encodes multiple enzymes, including an RNA-directed DNA polymerase reverse transcriptase, which transcribes a viral RNA into a double-stranded DNA, an integrase, which integrates a DNA produced by a reverse transcriptase into a host chromosomal DNA, and a protease, which processes encoded gag and pol genes. 5' and 3' LTRs promote the transcription and polyadenylation of virion RNA. In the production of a recombinant retroviral vector, gag, pol and env genes can be provided in trans by additional nucleic acid molecules (such as plasmids), so that these genes in a retroviral genome can be deleted, and are replaced with expression cassettes containing nucleic acids of interest, while gag, pol and env genes are provided in trans on one or more additional nucleic acid molecules (such as plasmids) to produce a recombinant retroviral vector.

The "recombinant retroviral vector" of the present invention refers to a retroviral particle that contains a structural and/or functional nucleic acid element derived from a retrovirus and is capable of transferring a nucleic acid of interest contained in the viral genome thereof into a cell, generally containing a retroviral capsid, a recombinant retroviral genome packaged in the capsid, and a viral envelope glycoprotein, wherein the recombinant retroviral genome contains an expression cassette for expressing the first chain or the second chain of the polypeptide molecule of the present invention. As is known to a person skilled in the art, a recombinant retroviral genome also contains at least long terminal repeat sequences (5' LTR and 3' LTR) at both ends of the genome and a sequence of packaging signal Ψ, and can optionally further contain a polypurine tract PPT. The recombinant retroviral vector of the present invention, for example, can be derived from Moloney murine leukaemia virus (MMLV) or murine stem cell virus (MSCV). The recombinant retroviral vector can be replication-competent or replication-defective. Typically, retroviral vectors are replication-defective, in which gene encoding regions necessary for the replication and packaging of viral particles are deleted or replaced with other genes. Therefore, once the initial target cell is infected, the virus cannot continue the typical cytolytic pathway thereof. This retroviral vector and a substance (for example, a packaging cell line) necessary for the production of this virus are commercially available (for example, available from Clontech).

A heterologous envelope glycoprotein (for example, an envelope glycoprotein from other viruses) can be used in a recombinant retroviral vector, that is, a heterologous env gene (for example, an env gene from other viruses) can be used in the production of the recombinant retroviral vector, to change the tropism thereof. For example, a VSV-G envelope glycoprotein or Sindbis virus E2 glycoprotein can be used. A recombinant retroviral vector packaged by using a heterologous envelope glycoprotein can also be referred to as a pseudotyped recombinant retroviral vector.

Methods for producing a recombinant retroviral vector are known to a person skilled in the art. For example, a packaging cell line can be provided with a nucleic acid molecule containing a recombinant retroviral genome, and one or more (such as two) nucleic acid molecules containing gag, pol and env genes, thereby producing a recombinant retroviral vector. The nucleic acid molecule can be a plasmid. The gag, pol and env genes can be provided by using two separate plasmids, wherein one plasmid contains gag and pol genes separately, and the other plasmid contains env gene, so that no recombination occurs between the plasmids. The recombinant retroviral genome contains an expression cassette (in which a nucleic acid of interest, such as the first expression cassette or the second expression cassette of the present invention, is contained) and cis-nucleic acid elements, such as long terminal repeat sequences (5' LTR and 3' LTR) and a sequence of packaging signal Ψ, necessary for the production of a recombinant retrovirus.

In the present invention, the first recombinant viral vector and the second recombinant viral vector can be derived from the same type of retrovirus or different types of retroviruses, and when both are derived from the same type of retrovirus, they can contain the same or different viral elements, such as the same or different 5' LTRs, 3' LTRs, sequences of packaging signal Ψ, gag, pol and/or env genes.

### Recombinant lentiviral vector

The recombinant viral vector of the present invention (such as the first recombinant viral vector and/or the second recombinant viral vector) can be a recombinant lentiviral vector. Lentivirus belongs to a retrovirus. Different from other retroviruses, lentivirus can be integrated into the genome of a non-dividing cell. Typical lentiviruses include human immunodeficiency virus (HIV), simian immunodeficiency virus (SIV) and feline immunodeficiency virus (FIV). Similar to other retroviruses, a lentiviral genome is flanked by two long terminal repeat (LTR) sequences, and contains gag, pol and env genes, which are the major genes encoding viral proteins, with a sequence of packaging signal Ψ between a 5' LTR and gag gene. In addition, other helper genes, including tat, rev, nef, vif, vpr and vpu, involved in synthesis and regulation, viral RNA processing and other replication functions are present in lentivirus. In the production of a recombinant lentiviral vector, nef, vif, vpr and vpu are not necessary, tat gene can no longer be necessary by modifying 5' LTR, and gag, pol, env and rev genes can be provided in trans by additional nucleic acid molecules (such as plasmids). Therefore, these genes in a lentiviral genome can be deleted and replaced with an expression cassette containing nucleic acids of interest, while gag, pol, env and rev genes are provided in trans on one or more additional nucleic acid molecules (such as plasmids), thereby producing a recombinant lentiviral vector.

The "recombinant lentiviral vector" of the present invention refers to a lentiviral particle that contains a structural and/or functional nucleic acid element derived from a lentivirus and is capable of transferring a nucleic acid of interest contained in the viral genome thereof into a cell, generally containing a lentiviral capsid, a recombinant lentiviral genome packaged in the capsid, and a viral envelope glycoprotein, wherein the recombinant lentiviral genome contains an expression cassette for expressing the first chain or the second chain of the polypeptide molecule of the present invention. As is known to a person skilled in the art, a recombinant lentiviral genome also contains at least long terminal repeat sequences (5' LTR and 3' LTR) at both ends of the genome and a sequence of packaging signal Ψ, and can optionally further contain a Rev-response element (RRE) and/or a DNA flap element (cPPT/CTS), etc. The recombinant lentiviral vector of the present invention can be derived from HIV-1, HIV-2, SIV or FIV. The recombinant lentiviral vector can be replication-competent or replication-defective. Typically, recombinant lentiviral vectors are replication-defective, in which gene encoding regions necessary for the replication and packaging of viral particles are deleted or replaced with other genes. Therefore, once the initial target cell is infected, the virus cannot continue the typical cytolytic pathway thereof.

The 3' LTR in the recombinant lentiviral vector of the present invention can be self-inactivating (SIN 3' LTR), for example, a U3 region is deleted, such that the lentiviral vector is incapable of self-replication. The U3 region of the 5' LTR in the recombinant lentiviral vector of the present invention can be replaced with a heterologous Tat-independent promoter, for example, promoters from other viruses, such as an RSV promoter, so that the dependence on Tat protein during transcription with U3 can be eliminated, and the Tat sequence can be removed during the production of a recombinant lentiviral vector.

A heterologous envelope glycoprotein (for example, an envelope glycoprotein from other viruses) can be used in a recombinant lentiviral vector, that is, a heterologous env gene (for example, an env gene from other viruses) can be used in the production of the recombinant lentiviral vector, to change the tropism thereof. For example, a VSV-G envelope glycoprotein or Sindbis virus E2 glycoprotein can be used. A recombinant lentiviral vector packaged by using a heterologous envelope glycoprotein can also be referred to as a pseudotyped recombinant lentiviral vector.

The production of a recombinant lentiviral vector is known to a person skilled in the art. For example, a packaging cell line can be provided with a nucleic acid molecule containing a recombinant lentiviral genome, one or more (such as two) nucleic acid molecules containing gag, pol and rev genes, and a nucleic acid molecule containing env gene, thereby producing a recombinant lentiviral vector. A recombinant lentiviral vector can be produced by using a three-plasmid or four-plasmid system. In a four-plasmid system, the first plasmid is deleted of helper proteins (such as tat, vif, vpu, vpr and nef), and contains gag and pol genes, the second plasmid contains rev gene, the third plasmid contains env gene of herpetic stomatitis virus envelope protein (VSV-G), and the fourth plasmid contains a recombinant lentiviral genome. Recombinant lentiviral vectors and systems for producing viruses are commercially available (such as available from Cell Biolabs, Inc.). The recombinant lentiviral genome contains an expression cassette (in which a nucleic acid of interest, such as the first expression cassette or the second expression cassette of the present invention, is contained) and cis-nucleic acid elements (such as long terminal repeat sequences (5' LTR and 3' LTR) and a sequence of packaging signal Ψ) necessary for the production of a recombinant lentiviral, and can also optionally contain a Rev-response element (RRE) and/or a DNA flap element (cPPT/CTS), etc.

In the present invention, the first recombinant viral vector and the second recombinant viral vector can be derived from the same type of lentivirus or different types of lentiviruses, and when both are derived from the same type of lentivirus, they can contain the same or different viral elements, such as the same or different 5' LTRs, 3' LTRs, sequences of packaging signal Ψ, gag, pol, rev and/or env genes.

### Recombinant HSV vector

The recombinant viral vector of the present invention (such as the first recombinant viral vector and/or the second recombinant viral vector) can be a recombinant HSV vector. Herpes simplex virus (HSV) is an enveloped double-stranded DNA virus with capsid, and the genome thereof comprises immediate early genes, early genes and late genes. HSV has two serotypes: HSV-1 and HSV-2. The replication of HSV virus in a host cell indicates lytic infection, which has the potential to cause the death of the host cell, so some immediate early genes (such as ICP4 and/or ICP27) are generally knocked out from recombinant HSV, rendering the virus replication-defective, and the virus can only regain the replication competence when the cell complements ICP4 and/or ICP27. Therefore, the recombinant HSV vector can be replication-defective.

The "recombinant HSV vector" of the present invention refers to an HSV particle containing a structural and/or functional nucleic acid element derived from HSV and is capable of transferring a nucleic acid of interest contained in the viral genome thereof into a cell, generally containing an HSV capsid, a recombinant HSV genome packaged in the capsid, and a viral envelope glycoprotein, wherein the recombinant HSV genome contains an expression cassette for expressing the first chain or the second chain of the polypeptide molecule of the present invention.

The recombinant HSV vector can be prepared by homologous recombination between an HSV backbone plasmid and a plasmid carrying a nucleic acid of interest. For example, generally, an HSV backbone plasmid contains the major functional genes of HSV, but if some immediate early genes, such as ICP4 and/or ICP27, are deleted, the HSV backbone plasmid and a plasmid carrying a nucleic acid of interest (for example, an expression cassette containing a nucleic acid of interest, for example, the first expression cassette or the second expression cassette of the present invention) are co-transfected into a production cell line expressing ICP4 and/or ICP27 and then homologous recombination occurs. Examples of production cell lines include, but are not limited to, a Vero cell.

In the present invention, the first recombinant viral vector and the second recombinant viral vector can be derived from the same type of HSV or different types of HSVs, and when both are derived from the same type of HSV, they can contain the same or different viral elements.

### Production of polypeptide molecule

The dual recombinant viral vector system of the present invention can be used for the production of the polypeptide molecule in vitro, for example, a host cell can be transduced with the first recombinant viral vector and the second recombinant viral vector contained in the dual recombinant viral vector system of the present invention, and is cultured under suitable conditions, such that the polypeptide molecule is expressed in the host cell. When the first recombinant viral vector and the second recombinant viral vector are provided in the form of a composition, the host cell can be transduced with a composition containing the first recombinant viral vector and the second recombinant viral vector. When the first recombinant viral vector and the second recombinant viral vector are provided separately, the host cell can be transduced first with one recombinant viral vector and then with another recombinant viral vector. The order of transduction can be arbitrary, and the host cell can be transduced with the first recombinant viral vector first or with the second recombinant viral vector first. The suitable conditions can be obtained by a person skilled in the art and are generally conditions suitable for the growth of the host cell. The first recombinant viral vector and the second recombinant viral vector express the first chain and second chain of the polypeptide molecule in the host cell, and the first and second chains can be spontaneously assembled into the polypeptide molecule. The host cell is in vitro. The polypeptide molecule can be collected and purified from the host cell culture for further use, or the host cell for expressing the polypeptide molecule can be applied for further use, for example, infusion into a subject for a therapeutic purpose.

The produced polypeptide molecule can be secreted extracellularly, and the secreted polypeptide molecule can be collected from a host cell culture supernatant. If necessary, the polypeptide molecule can be further purified by using methods well known in the art.

The term "transduction" used in the present invention refers to infecting a cell with a viral particle and having a function within the cell.

The term "host cell" of the present invention refers to a cell that contains a nucleic acid of interest and in which a polypeptide encoded by the nucleic acid of interest is expressed, including a cell into which an exogenous nucleic acid (such as the recombinant viral vector of the present invention) is introduced and the progeny thereof. The host cells can be isolated or in vitro. Examples of the host cells include bacteria, yeasts, mammalian cells, etc. Suitable host cells include, but are not limited to, insect cells such as Spodoptera frugiperda cells; microorganisms including bacteria, yeast cells such as Saccharomyces cerevisiae or methylotrophic yeast (Pichiapastoris), and fungi such as Trichoderma reesei, Aspergillus, Aureobasidum and Penicillium species; and mammalian cells such as Chinese hamster ovary (CHO) cells, baby hamster kidney (BHK) cells, COS cells, African green monkey kidney cells (Vero cells), canine kidney cells (such as MDCK cells), rat pituitary cells (GH1 cells), and human cell lines, including human embryonic kidney cells (such as HEK293, HEK293A, HEK293T and HEK293FT), Hela cells or hARPE-19 cells, etc.

A person skilled in the art knows how to select a suitable host cell according to different types of recombinant viral vectors used.

The dual recombinant viral vector system of the present invention can also be used for the production of the polypeptide molecule in vivo, for example, the polypeptide molecule can be produced in a subject, that is, the polypeptide molecule is delivered to a subject. The first recombinant viral vector and the second recombinant viral vector, after being delivered to a subject, can transduce cells in the subject, such as cells of tissues and/or organs, so as to express the first chain and second chain of the polypeptide molecule in the cells, tissues and/or organs. The first chain and second chain of the polypeptide molecule can be spontaneously assembled, after being expressed, into the polypeptide molecule, thereby producing the desired effect, such as a therapeutic effect, on a subject.

The dual recombinant viral vector system of the present invention can be specifically expressed in a specific cell, tissue and/or organ through the tropism of recombinant viral vectors and/or tissue-specific promoters, etc., achieving the purpose of targeted therapy. The produced polypeptide molecule can be secreted extracellularly, for example, the polypeptide molecule can be detected in serum.

The recombinant virus of the present invention can express a target antibody in the serum of a subject in an amount of at least approximately 9 µg/ml, at least approximately 10 µg/ml, at least approximately 50 µg/ml, at least approximately 100 µg/ml, at least approximately 200 µg/ml, at least approximately 300 µg/ml, at least approximately 400 µg/ml, at least approximately 500 µg/ml, at least approximately 600 µg/ml, at least approximately 700 µg/ml, at least approximately 800 µg/ml, at least approximately 900 µg/ml, or at least approximately 1000 µg/ml.

The "subject" of the present invention refers to an animal (including human) in need of alleviation, prevention and/or treatment of a disease. In some embodiments, the "subject" refers to a mammal, including, but not limited to, human, mouse, rat, rabbit, guinea pig, cow, horse, cat, dog, rodent, or primate. In some embodiments, the subject is a human. In some embodiments, the subject is a non-human mammal.

### Pharmaceutical composition

The first recombinant viral vector and the second recombinant viral vector in the dual recombinant viral vector system of the present invention can be provided separately, or provided in the form of a composition. The first recombinant viral vector and/or the second recombinant viral vector can be provided in the form of a pharmaceutical composition, and the pharmaceutical composition contains an effective amount of the first recombinant viral vector and/or the second recombinant viral vector, and optionally a pharmaceutically acceptable carrier. As is well known in the art, "pharmaceutically acceptable carrier" is a relatively inert substance that facilitates the administration of an active ingredient and enables a pharmaceutical composition to be provided in different dosage forms, such as a liquid solution, a suspension and an emulsion, or as a solid form suitable for dissolution or suspension in a liquid prior to use.

"Pharmaceutically acceptable carrier" includes, but is not limited to, a diluent, a filler, an antioxidant, a preservative, a stabilizer, an osmotic pressure regulator, a buffer and/or an emulsifier, etc. According to the route of administration, pharmaceutically acceptable carriers include, but are not limited to, sugars, starches, cellulose and derivatives thereof, maltose, gelatine, talc, calcium sulphate, vegetable oils, synthetic oils, polyols, alginic acids, phosphate buffer solutions, emulsifiers, isotonic saline and/or pyrogen-free water, etc. The pharmaceutical composition provided by the present invention can be prepared into clinically acceptable dosage forms such as a capsule, a tablet, a lozenge, a suppository, an ointment, a lotion, a paste, an aerosol, a spray, a gel, a patch and an injection.

### Delivery

The dual recombinant viral vector system or the pharmaceutical composition of the present invention can be delivered to a subject by using any suitable route, for example, systemic delivery or topical delivery, such as by oral, rectal, transmucosal, intranasal, inhalation, vaginal, intrathecal, intracerebroventricular, intraocular, and parenteral (such as intravenous, subcutaneous, intradermal, intramuscular and intraperitoneal) administration.

The dual recombinant viral vector systems or the pharmaceutical composition of the present invention can be delivered to any type of cells, including but not limited to nerve cells (including cells of peripheral and central nervous systems, such as neuronal cells and dendritic cells), lung cells, eye cells (including retinal cells, retinal pigment epithelium, and corneal cells), epithelial cells (such as intestinal and respiratory epithelial cells), muscle cells (such as skeletal muscle cells, cardiac muscle cells, smooth muscle cells and diaphragmatic muscle cells), liver cells, kidney cells, cancer cells, tumour cells, etc.

The dual recombinant viral vector system or the pharmaceutical composition of the present invention can be delivered to any tissue or organ, including but not limited to blood vessels, lungs, skin, heart, eyes (including vitreous and retina, particularly retinal pigment epithelium (RPE)), muscles (including skeletal muscle, cardiac muscle, smooth muscle and diaphragm), central nervous system (including spinal cord, brain parenchyma and cerebrospinal fluid) and liver, and can also be delivered to tumours (including tumours or lymph nodes).

In any given case, the most suitable route will depend on the nature and severity of the condition being treated and the properties of the specific viral vector being used. In term of inj ection, for example, a recombinant viral vector can be delivered to the brain by using any suitable device, such as a syringe, a needle, a cannula or catheter, or by stereotactic injection.

A single administration or more administrations (such as two, three, four or more administrations) of the dual recombinant viral vector system of the present invention can be performed on a subject to achieve the required level of the expression of the polypeptide molecule. In some embodiments, the first and second recombinant viral vectors can be administered to a subject once a year. In some embodiments, the first and second recombinant viral vectors are administered to a subject up to once every 5 years. In some embodiments, the first and second recombinant viral vectors are administered to a subject up to once every 10 years.

The doses of the first and second recombinant viral vectors can be adjusted by a person skilled in the art, for example, depending on the conditions of diseases, subjects and treatment schedules. The first and second recombinant viral vectors are generally administered in a "therapeutically effective amount", and the "treatment" of the present invention refers to preventing or alleviating (such as reducing, relieving or curing) at least one symptom associated with a disease state. The "therapeutically effective amount" of the present invention refers to an amount sufficient to prevent or alleviate (such as reduce, relieve or cure) at least one symptom associated with a disease state. The total dose and the fractionated doses of the first and second recombinant viral vectors administered to a subject depend on an administration method, a disease to be treated, the condition of an individual subject, specific viral vectors and a polypeptide molecule to be delivered, and can be determined in a conventional manner. The unit of dose, for example, can be vg/kg body weight.

### Disease to be treated

The dual recombinant viral vector system and method of the present invention can be used to produce a polypeptide molecule in vitro and apply the produced polypeptide molecule to the treatment of diseases, or the dual recombinant viral vector system is delivered to a subject so as to express the polypeptide molecule in the cell, tissue and organ of the subject, thereby preventing or treating one or more diseases in the subject.

The diseases to be prevented or treated depend on the specific polypeptide molecule expressed and the specific site of delivery. The diseases include, but are not limited to, viral infections, tumours, diseases associated with angiogenesis, etc. Examples of viral infections include, but are not limited to, infections caused by viruses selected from: coronavirus, filovirus, flavivirus, Norwalk virus, orthomyxovirus, paramyxoviridae, mumps virus, measles virus, parvovirus, small RNA virus, foot-and-mouth disease virus, enterovirus, coxsackie virus, poliovirus, rhinovirus, poxviridae, reoviridae, rotavirus, HIV virus, leukaemia virus, rubella virus, hepatitis C virus (HCV), hepatitis B virus, Epstein Barr virus, influenza virus, human immunodeficiency virus (HIV), respiratory syncytial virus and any combination thereof.

Examples of tumours include, but are not limited to, haematological tumours and solid tumours, wherein haematological tumours include, but are not limited to, myeloma, leukaemia, lymphoma, etc., and solid tumours include, but are not limited to, colorectal cancer, non-small cell lung cancer, small cell lung cancer, ovarian cancer, breast cancer, pancreatic cancer, gastric cancer, bladder cancer, oesophageal cancer, mesothelioma, melanoma, head and neck cancer, Hodgkin's lymphoma, hepatocellular carcinoma, advanced kidney cancer, prostate cancer, thyroid cancer, ovarian cancer, etc.

Examples of diseases associated with angiogenesis include, but are not limited to, macular degeneration (including age-related macular degeneration), arthritis (including rheumatoid arthritis), arteriosclerosis, diabetic retinopathy, haemangioma, inflammatory diseases, etc. The dual recombinant viral vector system of the present invention can also be used for treating autoimmune diseases such as multiple sclerosis.

Unless otherwise stated, the methods and materials in the examples described below are conventional products that can be purchased from the market. A person skilled in the art of the present invention would understand that the methods and materials described below are exemplary only and should not be considered as limiting the scope of the present invention.

### Example 1 Comparison of Avastin (bevacizumab) expression mediated by dual rAAV vector with that mediated by single rAAV vector in HEK293 cells and hARPE-19 cells

### A. Materials and methods

### 1. Design and production of AAV vector

The schematic diagrams of a vector expressing a complete antibody sequence (an AAV-Avastin single vector), a vector expressing a light chain only (an AAV-Avastin light chain, i.e., AAV-Avastin-L), and a vector expressing a heavy chain only (and an AAV-Avastin heavy chain, i.e., AAV-Avastin-H) are as shown in FIG. 1a, and transfer plasmid pAAV2.CMV.Avastin expressing a complete antibody sequence and transfer plasmids pAAV2.CMV.Avastin-L and pAAV2.CMV.Avastin-H expressing only a light chain or a heavy chain, respectively contain two ITRs and elements therebetween as shown in FIG. 1a. In the design of a single vector for AAV-mediated antibody expression, an F2A self-cleaving peptide motif is inserted between sequences encoding a heavy chain and a light chain to achieve the simultaneous expression of two polypeptides under the control of one promoter (see, such as US 20110065779 A1, US 9527904 B2, US 10093947 B2 and US 20180155412 A1).

Recombinant AAV was prepared by transfecting a 293FT cell with three plasmids: pAAV.R2C2 plasmid (i.e., pRC plasmid) expressing AAV2 *rep* and *cap* genes, pAdDelta6 plasmid (i.e., pHelper plasmid) expressing a helper gene from adenovirus and a transfer plasmid, see Xiao and Samulski, 1998, J Virol., wherein the pAdDelta6 plasmid was constructed by synthesizing DNA independently with reference to Addgene plasmid # 112867 sequence, the pAAV.R2C2 plasmid was constructed from Addgene plasmid # 112864 pAAV.R2C8 by removing AAV8 *cap* gene and inserting AAV2 *cap* gene, with the genome sequence of AAV2 referring to GenBank: AF043303.

Construction of single vector transfer plasmid: the gene of a target antibody (Avastin) was designed and synthesized by sequentially linking a heavy chain leader sequence, a heavy chain, F2A, a light chain leader sequence and a light chain in series; Not I and Hind III restriction sites were added in the upstream and downstream, respectively; the gene was constructed between the promoter of an AAV expression vector pAAV.CMV.PI.EGFP.WPRE.bGH (constructed by synthesizing DNA independently with reference to Addgene plasmid # 105530) and poly A by enzyme digestion and linking; the transfer plasmid pAAV2.CMV.Avastin of the recombinant AAV antibody was obtained by transformation, cloning and amplification; and the target sequence alignment by Sanger sequencing showed that the inserted sequence was completely correct. The sequences used therein were as follows, with the Avastin sequence referring to https://go.drugbank.com/drugs/DB00112:

| **Single vector** | |
|---|---|
| **Name** | **Amino acid sequence** |
| Avastin heavy chain | EVQLVESGGGLVQPGGSLRLSCAASGYTFTNYGMNWVRQAPGKGLEWVGWINTYTGEPTY AADFKRRFTFSLDTSKSTAYLQMNSLRAEDTAVYYCAKYPHYYGSSHWYFDVWGQGTLVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS REEMTKNQVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 19) |
| Avastin light chain | DIQMTQSPSSLSASVGDRVTITCSASQDISNYLNWYQQKPGKAPKVLIYFTSSLHSGVPS RFSGSGSGTDFTLTISSLQPEDFATYYCQQYSTVPWTFGQGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC (SEQ ID NO: 20) |

| **Name** | **Nucleotide sequence** |
|---|---|
| Heavy chain leader sequence | ATGGGCTGGTCCTGCATCATCCTGTTTCTGGTGGCCACCGCTACAG GAGTGCACTCC (SEQ ID NO: 1) |
| Heavy chain | GAGGTGCAGCTGGTCGAATCCGGCGGAGGCCTGGTGCAGCCCGGC GGGTCTCTGCGGCTGTCTTGTGCCGCTTCTGGCTACACCTTCACCA |

| | |
|---|---|
| | ACTACGGCATGAATTGGGTCAGACAGGCCCCTGGCAAGGGCCTGG AATGGGTGGGCTGGATCAACACCTATACCGGCGAGCCCACCTACGC CGCCGACTTCAAGCGGCGGTTCACGTTCAGCCTGGATACCTCCAA ATCTACAGCCTACCTGCAGATGAACTCCCTGAGAGCCGAGGACAC CGCTGTGTACTACTGCGCCAAGTACCCTCACTACTACGGCTCTTCT CACTGGTATTTCGACGTGTGGGGCCAGGGAACACTGGTCACAGTG TCCTCTGCCTCTACCAAGGGCCCTTCTGTGTTTCCTCTGGCCCCTA GCAGCAAGTCCACCTCCGGCGGCACCGCCGCCCTGGGCTGCCTGG TGAAGGACTACTTCCCAGAACCTGTGACCGTGTCTTGGAACTCCG GCGCTCTGACCTCTGGCGTGCACACATTTCCAGCTGTTCTGCAGTC CTCCGGACTGTACTCTCTGTCCTCCGTTGTGACCGTGCCTTCCAGC AGCTTGGGCACACAGACCTACATCTGCAACGTGAACCACAAGCCT TCTAACACCAAAGTGGATAAGAAGGTGGAACCTAAGTCCTGCGAC AAGACCCACACCTGCCCTCCTTGTCCTGCTCCTGAGCTGCTGGGC GGACCTTCTGTGTTCCTGTTCCCTCCTAAGCCCAAGGACACCCTGA TGATCTCTAGAACCCCCGAGGTGACATGCGTGGTCGTGGACGTGTC TCACGAGGATCCTGAAGTGAAGTTCAACTGGTACGTGGACGGCGT GGAAGTGCACAACGCCAAGACCAAGCCTAGAGAGGAACAGTACA ACTCTACCTACAGAGTGGTGTCCGTGCTGACCGTGCTGCACCAGG ACTGGCTGAACGGCAAAGAGTACAAGTGCAAGGTGTCCAACAAG GCTCTGCCTGCTCCTATCGAGAAGACCATCTCCAAGGCCAAAGGC CAGCCTCGCGAGCCTCAAGTGTACACCCTGCCCCCATCTCGGGAA GAGATGACCAAGAACCAGGTGTCTCTAACCTGTCTGGTCAAGGGC TTCTACCCCTCCGACATCGCCGTGGAGTGGGAGTCCAACGGCCAG CCTGAGAACAACTACAAAACCACCCCTCCCGTGCTGGACTCCGAT GGCTCCTTCTTCCTGTACTCCAAGCTGACCGTGGACAAGTCCAGAT GGCAGCAGGGCAACGTGTTCTCCTGCTCCGTGATGCACGAGGCCC TGCATAATCACTACACCCAGAAGTCTCTGTCCCTGTCTCCTGGCAA G (SEQ ID NO: 2) |
| F2A | GGCAGCGGCGTGAAGCAGACCCTGAACTTCGACCTGCTGAAGCTG GCCGGCGACGTGGAGAGCAACCCCGGCCCC (SEQ ID NO: 3) |
| Light chain leader sequence | ATGGGCTGGTCCTGCATCATCCTGTTCCTGGTGGCTACCGCTACCG GCGTGCACTCT (SEQ ID NO: 4) |
| Light chain | GATATCCAGATGACCCAGTCTCCCAGCTCTCTGTCTGCTTCTGTGG GCGACAGAGTGACCATCACCTGTTCTGCCTCTCAGGACATCTCCAA CTACCTGAACTGGTACCAGCAGAAGCCTGGCAAGGCTCCTAAAGT GCTGATCTACTTCACCTCCAGCCTGCACTCCGGAGTGCCCAGCCGG TTCTCCGGCTCTGGCTCCGGCACCGACTTCACCCTGACAATCTCTT CTCTGCAGCCTGAGGACTTCGCCACCTACTACTGCCAGCAGTACTC TACCGTGCCTTGGACCTTCGGCCAGGGAACCAAAGTGGAAATCAA GCGGACCGTGGCCGCTCCATCCGTGTTCATCTTCCCTCCTTCCGAC GAACAGCTGAAGTCTGGCACAGCCTCCGTGGTGTGCCTGCTGAAC AACTTCTACCCTAGAGAGGCCAAGGTGCAGTGGAAGGTGGACAAC GCCCTGCAGTCCGGCAACTCCCAAGAGTCCGTCACCGAGCAGGAT TCCAAGGACTCCACCTACTCCCTGTCCTCCACACTGACCCTGTCTA |
| | AGGCCGACTACGAGAAGCACAAGGTGTACGCCTGCGAGGTGACC CACCAGGGCCTGAGCTCTCCTGTGACCAAGTCCTTCAACAGAGGC GAGTGTtga (SEQ ID NO: 5) |

Construction of dual vector transfer plasmid (comprising heavy chain transfer plasmid and light chain transfer plasmid):
Heavy chain transfer plasmid: the heavy chain gene of a target antibody (Avastin) was designed and synthesized according to the sequence of a heavy chain leader sequence and a heavy chain; Not I and Hind III restriction sites were added in the upstream and downstream, respectively; the heavy chain gene was constructed between the promoter of an AAV expression vector pAAV.CMV.PI.EGFP.WPRE.bGH and poly A by enzyme digestion and linking; the transfer plasmid pAAV2.CMV.Avastin-H of the recombinant AAV antibody heavy chain was obtained by transformation, cloning and amplification; and the target sequence alignment by Sanger sequencing showed that the inserted sequence was completely correct.

The heavy chain leader sequence and heavy chain sequence in the heavy chain transfer plasmid were identical to those in the single vector transfer plasmid, respectively.

Light chain transfer plasmid: the light chain gene of a target antibody (Avastin) was designed and synthesized according to the sequence of a light chain leader sequence and a light chain; Not I and Hind III restriction sites were added in the upstream and downstream, respectively; the light chain gene was constructed between the promoter of an AAV expression vector pAAV.CMV.PI.EGFP.WPRE.bGH and poly A by enzyme digestion and linking; the transfer plasmid pAAV2.CMV.Avastin-L of the recombinant AAV antibody light chain was obtained by transformation, cloning and amplification; and the target sequence alignment by Sanger sequencing showed that the inserted sequence was completely correct.

The light chain leader sequence and light chain sequence in the light chain transfer plasmid were identical to those in the single vector transfer plasmid, respectively.

pAAV.R2C2, pAdDelta6 and each of the above-mentioned three transfer plasmids were co-transfected into 2E+08 HEK293FT cells in a total amount of 1040 µg DNA and a molar ratio of 1 : 1 : 1. The recombinant AAV vector was harvested 48 hours after transfection. Cells were detached from an adherent plate with 0.25% Trypsin-EDTA and lysed by freezing and thawing three times. After centrifuging at 12,000 rpm for 8 minutes, the supernatant was collected to obtain the recombinant AAV vectors AAV2-Avastin (single vector) or AAV2-Avastin-L (the light chain vector in the dual vectors) and AAV2-Avastin-H (the heavy chain vector in the dual vectors), respectively. The viral genome titre of the crude cell lysate of each recombinant AAV vector was determined by droplet digital PCR (ddPCR).

### 2. Vector transduction and antibody detection

HEK293FT cells were co-transduced with AAV2-Avastin or with both AAV2-Avastin-L and AAV2-Avastin-H, in which the ratio of AAV2-Avastin-L to AAV2-Avastin-H was 1 : 4, 1 : 2, 1 : 1, 2 : 1, 4 : 1 and 6 : 1 (based on a viral genome titre), respectively. A vector containing only a light chain or a vector containing only a heavy chain was also transduced for use as a control. 293FT cells were seeded in a 24-well plate at 1.2E+05 cells per well and cultured overnight in a DMEM/10% FBS complete medium at 37°C under 5% CO₂. The medium was removed. A total amount of 5.0E+10 vg of a viral vector diluted with the above-mentioned complete medium was added. After incubation for 2.5 hours in a 37°C, 5% CO₂ incubator, 1 mL of a fresh complete medium was added for continuing culture. The cell culture supernatant was collected 48 hours after transduction. Each sample was diluted with a loading buffer under a non-reducing condition (FIG. 1b) and a reducing condition (FIG. 1c), respectively, and Western blot was performed with a goat anti-human IgG antibody (Invitrogen, Cat No: A18811) to detect the expression of Avastin.

hARPE-19 cells were seeded in a 24-well plate at 8.0E+04 cells per well and cultured overnight in a F12/10% FBS medium at 37°C under 5% CO₂. The medium was removed. A total amount of 5.0E+10 vg of viral vectors, i.e., AAV2-Avastin, AAV2-Avastin-L and AAV2-Avastin-H, diluted with a F12/10% FBS medium were added, wherein AAV2-Avastin-L and AAV2Avastin-H were combined at a viral genome titre ratio of 1 : 4, 1 : 2, 1 : 1, 2 : 1 and 4:1. After incubation for 2.5 hours in a 37°C, 5% CO₂ incubator, 1 mL of a fresh complete medium was added for continuing culture. The cell culture supernatant was collected 48 hours after transduction. Each sample was diluted with a loading buffer under a non-reducing condition (FIG. 1d), and Western blot was performed with a goat anti-human IgG antibody (Invitrogen, Cat No: A18811) to detect the expression of Avastin.

In Western blot, the positive control was purified Avastin, and the negative control was a cell supernatant not transduced with a virus.

### B. Results

As shown in FIG. 1, both single vector transduction and dual vector transduction successfully produce Avastin secreted in HEK293FT cells (FIGs. 1b and 1c) and hARPE-19 cells (FIG. 1d). With the increase of the proportion of the light chain vector, the concentration of the antibody increases. Diagrams b and d show the electrophoresis in a loading non-reducing buffer, in which the heavy and light chains of Avastin are present in the form of a tetramer. Diagram c shows the electrophoresis in a loading reducing buffer, in which Avastin is dissociated, and the heavy chain and the light chain are present in the form of a single chain, respectively.

### Example 2 Avastin expression mediated by dual rAAV vector having other serotype capsid in cell line

### A. Materials and methods

In another embodiment, the capsid serotype of the AAV vector is AA8, AAV9, or AAV5. pAAV.R2C9 plasmid was constructed from Addgene plasmid # 112864 pAAV.R2C8 by removing AAV8 *cap* gene and inserting AAV9 *cap* gene, with the AAV9 *cap* gene sequence referring to GenBank: AY530579.1. pAAV.R2C5 plasmid was constructed from Addgene plasmid # 112864 pAAV.R2C8 by removing 1 AAV8 *cap* gene and inserting AAV5 *cap* gene, with the genome sequence of AAV5 referring to NCBI sequence: NC_006152. The packaging steps were the same as those in Materials and methods 1 of example 1, except that pAAV.R2C2 containing AAV genomic *rep* and *cap* genes was replaced with pAAV.R2C8, pAAV.R2C9 and pAAV.R2C5 plasmids containing the *cap* gene of the above-mentioned serotype, respectively. HEK293FT cells were co-transfected with pAdDelta6 and pAAV2.CMV.Avastin containing a complete antibody expression cassette or pAAV2.CMV.Avastin-H containing only an antibody heavy chain expression cassette or pAAV2.CMV.Avastin-L containing only an antibody light chain expression cassette. After 48 hours, the cell culture supernatant and the virus in the cells were harvested to obtain AAV8-Avastin, AAV8-Avastin-L and AAV8-Avastin-H having an AAV8 capsid, AAV9-Avastin, AAV9-Avastin-L and AAV9-Avastin-H having an AAV9 capsid, and AAV5-Avastin, AAV5-Avastin-L and AAV5-Avastin-H having an AAV5 capsid, respectively. The viral genome titres were determined by ddPCR.

HEK293FT cells were seeded in a 24-well plate at a density of 1.2E+05 cells per well, and 500 µL of a DMEM/10% FBS medium was added for culturing overnight; and hARPE-19 cells were seeded in a 24-well plate at a density of 8.0E+04 cells per well, and a F12/10% FBS medium was added for culturing overnight. After 24 hours, the mediums were removed, and recombinant AAV or a recombinant AAV combination with a total amount of approximately 1.3E+10 vg and a titre of 4.0E+10 vg/mL or with a total amount of approximately 3.8E+10 vg and a titre of 1.2E+11 vg/mL was added, respectively (the ratio based on a viral genome titre):
AAV8-Avastin,
AAV8-Avastin-L: AAV8-Avastin-H 4 : 1,
AAV8-Avastin-L: AAV8-Avastin-H 2 : 1,
AAV8-Avastin-L: AAV8-Avastin-H 1 : 1,
AAV8-Avastin-L: AAV8-Avastin-H 1 : 2,
AAV9-Avastin,
AAV9-Avastin-L: AAV9-Avastin-H 4 : 1,
AAV9-Avastin-L: AAV9-Avastin-H 2 : 1,
AAV9-Avastin-L: AAV9-Avastin-H 1 : 1,
AAV9-Avastin-L: AAV9-Avastin-H 1 : 2,
AAV5-Avastin,
AAV5-Avatin-L: AAV5-Avastin-H 4 : 1,
AAV5-Avatin-L: AAV5-Avastin-H 2 : 1,
AAV5-Avastin-L: AAV5-Avastin-H 1 : 1 or
AAV5-Avastin-L: AAV5-Avastin-H 1 : 2.

After incubation for 2.5 hours in a 37°C, 5% CO₂ incubator, 1 mL of fresh culture medium was added, and incubating was performed for another 67 hours. The cell culture supernatant was collected, and Western blot was performed with an anti-human IgG antibody to detect the expression of Avastin.

### B. Results

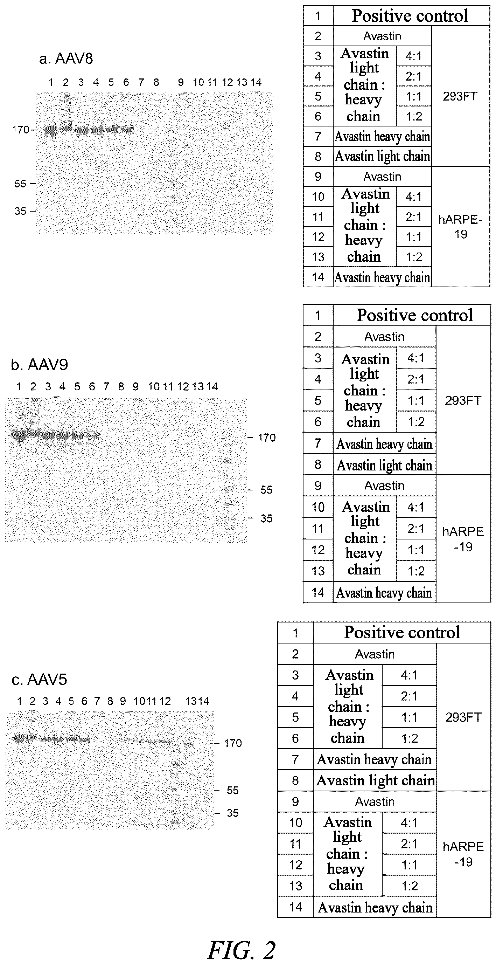

As shown in FIG. 2, Avastin can be expressed in HEK293FT cells or hARPE-19 cells transduced with a single vector or dual vectors having an AAV8 capsid (diagram a), an AAV9 capsid (diagram b) and an AAV5 capsid (diagram c), wherein the level of antibody expression mediated by the dual vectors having an AAV8 capsid and an AAV9 capsid is higher than that mediated by the single vector in 293FT cells, and with the increase of the proportion of the light chain vector, the expression level also increases. The dual vectors having an AAV5 capsid do not show a significant correlation of heavy/light chain ratio, but still have a higher expression level than the single vector, and have a stronger tropism for hAPRE-19 cells.

Avastin expressed by using the single vector has obvious bands with a molecular weight larger or smaller than that of the antibody itself, and the bands can be antibody single chains that are not correctly paired. The impure expression limits the use of AAV in the delivery of an antibody in vivo. With regard to the antibody expressed by using the dual vector system having an AAV8 capsid, an AAV9 capsid and an AAV5 capsid, the content of mispairing bands is significantly reduced.

### Example 3: Comparison of antibody expression by in vitro plasmid transfection and virus vector transduction

In another embodiment, the antibody expression efficiency of AAV vector was compared with that of plasmid vector.

### A. Materials and methods

According to the packaging steps in Materials and methods 1 of example 1, AAV2-Avastin, AAV2-Avastin-L and AAV2-Avastin-H were packaged and produced, and the viral genome titres were determined by using a ddPCR method. HEK293FT cells were seeded in a 24-well plate at a density of 2.0E+05 cells per well, and 500 µL of a DMEM/10% FBS medium was added. hARPE-19 cells were seeded at a density of 8.0E+04 cells per well, and a F12/10% FBS medium was added. Both were cultured overnight. After 24 hours, an AAV vector was transduced into HEK293FT cells at a total amount of 1.0E+10 vg, a titre of 3.3E+10 vg/mL and a volume of 300 µL per well. A transfer plasmid was respectively mixed with twice the mass of PEI for transfecting HEK293FT cells and hARPE-19 cells at a total mass of 0.1 µg or 1.0 µg (equivalent to 1.0E+10 copy number or 1.0E+1 1 copy number). The recombinant AAVs or the recombinant AAV combinations and the plasmid combinations were respectively:
AAV2-Avastin,
AAV2-Avastin-H,
AAV2-Avastin-L,
AAV2-Avastin-L: AAV2-Avastin-H 4 : 1,
AAV2-Avastin-L: AAV2-Avastin-H 2 : 1,
AAV2-Avastin-L: AAV2-Avastin-H 1 : 1,
pAAV2.CMV.Avastin,
pAAV2.CMV.Avastin-H,
pAAV2.CMV.Avastin-L, pAAV2.CMV.Avastin-L: pAAV2.CMV.Avastin-H 4 : 1,
pAAV2.CMV.Avastin-L: pAAV2.CMV.Avastin-H 2 : 1,
pAAV2.CMV.Avastin-L: pAAV2.CMV.Avastin-H 1 : 1,
pAAV2.CMV.Avastin-L: pAAV2.CMV.Avastin-H 1 : 2.

After incubation for 2.5 hours in a 37°C, 5% CO₂ incubator, 1 mL of fresh culture medium was added, and incubating was performed for another 67 hours. The cell culture supernatant was collected, and Western blot was performed with an anti-human IgG antibody to detect the expression of Avastin.

### B. Results

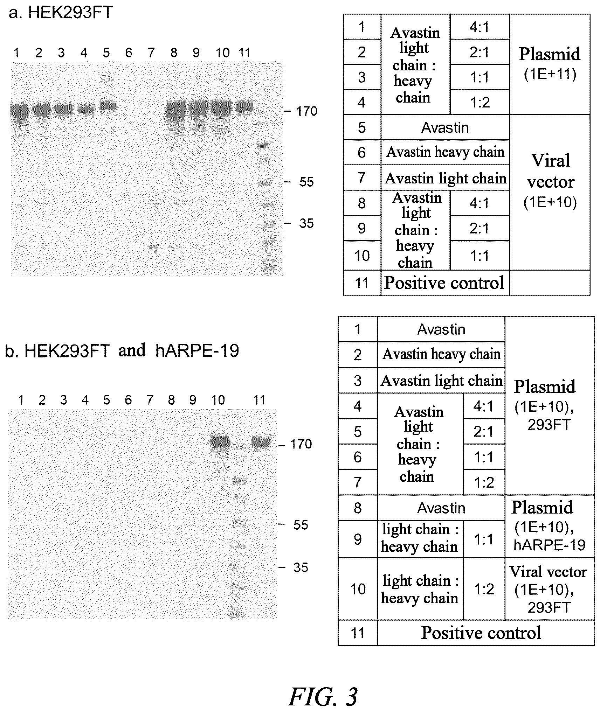

As shown in FIG. 3, the positive control is purified Avastin, with a loading amount of 20 ng. As shown in diagram a, antibodies (lanes 1-4) are expressed in cells transfected with plasmids having 10 times the number of gene copies of viral vectors, in which the expressions are still lower than those in cells transduced with the viral vectors (lanes 8-10). For the viral vectors, the expression of antibodies by using dual vectors is higher than that by using single vector. The concentration of the antibody in the dual viral vector supernatant is greater than 1 ng/µL, and with the increase of the proportion of the light chain, the expression level of the antibody increases. As shown in diagram b, when the genome copy number of the plasmid vector is similar to that of the viral vector, the concentration of the antibody in the supernatant is far lower than that of the viral vector (lane 12), that is, far lower than 1 ng/µL, regardless of the transfection of HEK293FT cells (lanes 1-7) or the transfection of hARPE-19 cells (lanes 8 and 9).

### Example 4 Avastin expression mediated by dual rAAV vector in hiPSC-RPE cells

In this example, antibodies were expressed in human induced pluripotent stem cell-derived retinal pigment epithelium (hiPSC-RPE) transduced with recombinant AAV dual vectors.

### A. Materials and methods

Retinal pigment epithelium precursors (RPE precursors, Nuwacell) were resuscitated and then serially cultured in a 6-well plate coated with Matrigel by using a medium containing RPE maintenance (Nuwacell, RP0106-H), Maintenance supplement (Nuwacell, RP01016-H), Blebbistatin, penicillin and streptomycin, wherein the medium was changed every 2 days. After 12 days, the cells were passaged once, seeded in a 12-well plate at a density of 1.0E+06 cells per well, and continued to be cultured in the above-mentioned medium, wherein the medium was changed every 2 days. 13 days after seeding, a total amount of 1.5E+10 vg of virus was added to transduce the cells according to the methods in examples 1 and 2, and the recombinant AAVs or the recombinant AAV combinations respectively were:
AAV2-Avastin,
AAV2-Avastin-H,
AAV2-Avastin-L,
AAV2-Avastin-L: AAV2-Avastin-H 4 : 1,
AAV2-Avastin-L: AAV2-Avastin-H 2 : 1,
AAV2-Avastin-L: AAV2-Avastin-H 1 : 1.

The culture supernatants were collected on days 2, 6, 12, 15 and 18 after transduction, and new medium was added. The Avastin in the supernatant was detected according to the Western blot method of examples 1-3, the Avastin positive control was loaded at 40 ng, a supernatant to be detected was loaded at 20 µL, and a loading buffer was a non-reducing buffer.

### B. Results

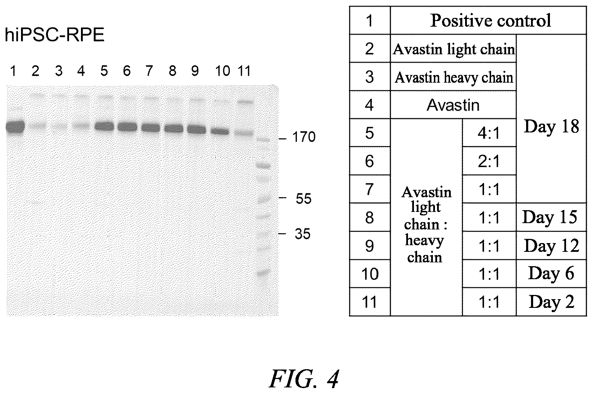

As shown in FIG. 4, the expression and secretion of Avastin in hiPSC-RPE cells increases significantly on day 2 after AAV transduction, and the secretion amount remains unchanged when detected on the 18th day. The expression of the antibody in cells transduced with dual AAV vectors is significantly better than that in cells transduced with a single AAV vector.

### Example 5 ABTAA expression mediated by dual rAAV vectors in 293 cells

In some embodiments, dual AAV vectors are used for delivering anti-Ang2 antibody, i.e., ABTAA.

### A. Materials and methods

Similar to the method for constructing Avastin transfer plasmid in example 1, schematic diagrams of a transfer plasmid pAAV2.CMV.ABTAA expressing a complete ABTAA sequence, a transfer plasmid pAAV2.CMV.ABTAA-L expressing only a light chain and a transfer plasmid pAAV2.CMV.ABTAA-H expressing only a heavy chain were as shown in FIG. 5a.

The gene of a target antibody (ABTAA) was designed and synthesized by sequentially linking a heavy chain leader sequence, a heavy chain, F2A, a light chain leader sequence and a light chain in series; Not I and Hind III restriction sites were added in the upstream and downstream, respectively; the gene was constructed between the promoter of an AAV expression vector pAAV.CMV.PI.EGFP.WPRE.bGH and poly A by enzyme digestion and linking; the transfer plasmid pAAV2.CMV.ABTAA of the AAV-ABTAA single vector was obtained by transformation, cloning and amplification. With reference to a heavy chain variable region (US 9,902,767 B2 SEQ ID NO: 7) and a light chain variable region (US 9,902,767 B2 SEQ ID NO: 9) of SAIT-ANG2-AB-m10D6 in US 9,902,767 B2, ABTAA was obtained by reverse translation and codon optimization, and was linked to a heavy chain constant region and a light chain constant region of human IgG4, respectively. The target sequence alignment by Sanger sequencing showed that the inserted sequence was completely correct. The sequences used therein were as follows:

| ABTAA | |
|---|---|
| Name | Amino acid sequence |
| Heavy chain variable region | DVQLQESGPGLVKPSQSLSLTCTVTGYSITSDYAWNWIRQFPGNK LEWMGYINYSGNTDYNPSLKSRSSITRDTSKNQFFLQLNSVTTGD TATYYCARGNFEGAMDYWGQGTSVTVSS (SEQ ID NO: 21) |
| Light chain variable region | SIVMTQTPKFLLVSAGDRVTITCKASQSVSNDVAWYQQKPGQSP KLLIYYASNRYPGVPDRFTGSGYGTDFTFTISTVQAEDLAVYFCQQ DYSSPWTFGGGTKLEIK (SEQ ID NO: 22) |

| Name | Nucleic acid sequence |
|---|---|
| Heavy chain leader chain | ATGGGCTGGTCCTGCATCATCCTGTTCCTGGTGGCCACCGCTACCGGA GTGCACTCC (SEQ ID NO: 6) |
| Heavy chain | GACGTGCAGCTGCAAGAGTCCGGCCCTGGCCTGGTGAAGCCTTCC CAGTCCCTGTCCCTGACCTGCACCGTGACCGGCTACTCCATCACCT CCGACTACGCCTGGAACTGGATCAGACAGTTCCCCGGCAACAAGC TGGAATGGATGGGCTACATCAACTACTCTGGAAACACCGACTACAA CCCTTCTCTGAAGTCCAGATCCTCTATCACCAGAGATACCTCCAAG AACCAGTTCTTCCTGCAGCTGAACTCCGTGACCACCGGCGACACC GCCACCTACTACTGCGCCAGAGGCAACTTCGAGGGCGCCATGGAC TACTGGGGCCAGGGCACCTCTGTGACAGTGTCCTCCGCCTCTACAA AGGGCCCCAGCGTTTTCCCACTGGCTCCCTGTAGCAGAAGCACCA GCGAATCTACAGCCGCTCTGGGCTGCCTGGTCAAGGACTACTTTCC TGAGCCTGTGACCGTGTCCTGGAACTCTGGCGCTCTGACAAGCGG |
| | AGTGCACACCTTTCCAGCCGTGCTGCAAAGCAGCGGCCTGTACTC TCTGAGCAGCGTGGTCACAGTGCCTAGCTCTAGCCTGGGCACCAA GACCTACACCTGTAATGTGGACCACAAGCCTAGCAACACCAAGGT GGACAAGCGCGTGGAATCTAAGTACGGCCCTCCTTGTCCTCCATGT CCTGCACCTGAGTTTCTCGGCGGACCCTCCGTGTTCCTGTTTCCTC CAAAGCCTAAGGATACCCTGATGATCAGCAGAACCCCTGAAGTGA CCTGCGTGGTGGTGGACGTGTCCCAAGAGGATCCTGAGGTGCAGT TCAATTGGTACGTGGACGGCGTGGAAGTGCACAACGCCAAGACCA AGCCTAGAGAGGAACAGTTCAACAGCACCTACAGAGTGGTGTCCG TGCTGACAGTGCTGCACCAGGATTGGCTGAACGGCAAAGAGTACA AGTGCAAGGTGTCCAACAAGGGCCTGCCTAGCAGCATCGAGAAAA CCATCAGCAAGGCCAAGGGCCAGCCAAGAGAACCCCAGGTGTAC ACACTGCCTCCAAGCCAAGAGGAAATGACCAAGAACCAGGTGTCC CTGACCTGCCTCGTGAAGGGATTCTACCCCTCCGATATCGCCGTGG AATGGGAGAGCAATGGACAGCCCGAGAACAACTACAAGACAACC CCTCCTGTGCTGGACAGCGACGGCTCATTCTTCCTGTACAGCAGAC TGACCGTGGACAAGAGCAGATGGCAAGAGGGCAACGTGTTCTCCT GCAGCGTGATGCACGAGGCCCTGCACAACCACTACACCCAGAAGT CTCTGTCCCTGTCTCTGGGCAAG (SEQ ID NO: 7) |
| F2A | GGCAGCGGCGTGAAGCAGACCCTGAACTTCGACCTGCTGAAGCTG GCCGGCGACGTGGAGAGCAACCCCGGCCCC (SEQ ID NO: 8) |
| Light chain leader chain | ATGGGCTGGTCCTGCATCATCCTGTTCCTGGTGGCCACCGCTACCG GAGTGCACTCC (SEQ ID NO: 9) |
| Light chain | TCCATCGTGATGACCCAGACCCCTAAGTTCCTGCTGGTGTCTGCTG GCGACAGAGTGACCATCACCTGCAAGGCCTCTCAGTCCGTGTCCA ACGACGTGGCCTGGTACCAGCAGAAGCCTGGCCAGTCTCCCAAGC TGCTGATCTACTACGCCTCCAACAGATACCCCGGCGTGCCTGACCG GTTCACCGGCTCTGGCTACGGCACCGACTTCACCTTTACAATCTCC ACCGTGCAGGCCGAGGACCTGGCCGTGTACTTCTGCCAGCAGGAC TACTCCTCTCCTTGGACCTTCGGCGGCGGAACCAAGCTGGAAATCA AGAGAACCGTGGCCGCCCCTAGCGTGTTCATCTTTCCTCCCTCTGA CGAGCAGCTGAAATCTGGCACCGCTTCCGTGGTCTGCCTGCTGAA CAACTTCTACCCCAGAGAGGCTAAGGTGCAGTGGAAAGTGGACAA CGCCCTGCAGTCTGGCAACTCCCAAGAAAGCGTGACCGAGCAGG ACTCCAAGGACTCTACATACAGCCTGTCCTCCACCCTGACCCTGTC CAAGGCCGATTACGAGAAGCACAAGGTCTACGCTTGTGAAGTGAC CCACCAGGGACTGTCCTCCCCTGTGACCAAGTCTTTCAATCGGGGC GAGTGCtga (SEQ ID NO: 10) |

Construction of dual vector transfer plasmid (comprising heavy chain transfer plasmid and light chain transfer plasmid), which is similar to the method of example 1:
Heavy chain transfer plasmid: the heavy chain gene of a target antibody (ABTAA) was designed and synthesized according to the sequence of a heavy chain leader sequence and a heavy chain; Not I and Hind III restriction sites were added in the upstream and downstream, respectively; the heavy chain gene was constructed between the promoter of an AAV expression vector pAAV.CMV.PI.EGFP.WPRE.bGH and poly A by enzyme digestion and linking; the transfer plasmid pAAV2.CMV.ABTAA-H of the AAV-ABTAA heavy chain was obtained by transformation, cloning and amplification; and the target sequence alignment by Sanger sequencing showed that the inserted sequence was completely correct.

The heavy chain leader sequence and heavy chain sequence in the heavy chain transfer plasmid were identical to those in the single vector transfer plasmid, respectively.

Light chain transfer plasmid: the light chain gene of a target antibody (ABTAA) was designed and synthesized according to the sequence of a light chain leader sequence and a light chain; Not I and Hind III restriction sites were added in the upstream and downstream, respectively; the light chain gene was constructed between the promoter of an AAV expression vector pAAV.CMV.PI.EGFP.WPRE.bGH and poly A by enzyme digestion and linking; the transfer plasmid pAAV2.CMV.ABTAA-L of the AAV-ABTAA light chain was obtained by transformation, cloning and amplification; and the target sequence alignment by Sanger sequencing showed that the inserted sequence was completely correct.

The light chain leader sequence and light chain sequence in the light chain transfer plasmid were identical to those in the single vector transfer plasmid, respectively.

Packaging and harvesting of the recombinant AAV were consistent with the method of example 1. pAAV.R2C2, pAdDelta6 and each of the above-mentioned three transfer plasmids were co-transfected into 2E+08 HEK293FT cells in a total amount of 1040 µg DNA and a molar ratio of 1 : 1 : 1. The recombinant AAV vector AAV2-ABTAA (single vector) or AAV2-ABTAA-L (the light chain vector in the dual vectors) and AAV2-ABTAA-H (the heavy chain vector in the dual vectors) were harvested 48 hours after transfection, and the viral genome titre of the crude cell lysate of each recombinant AAV vector was determined by droplet digital PCR (ddPCR).

### 2. Vector transduction and antibody detection

HEK293FT cells were transduced with AAV2-ABTAA or co-transduced with both AAV2-ABTAA-L and AAV2-ABTAA-H, in which the ratio of AAV2-ABTAA-L to AAV2-ABTAA-H was 1 : 4, 1 : 2, 1 : 1, 2 : 1 and 4 : 1 (based on a viral genome titre), respectively. A vector containing only a light chain or a vector containing only a heavy chain was also transduced for use as a control. 293FT cells were seeded in a 24-well plate at 1.2E+05 cells per well and cultured overnight in a DMEM/10% FBS complete medium at 37°C under 5% CO₂. The medium was removed. A total amount of 7.0E+09 vg of a viral vector diluted with the above-mentioned complete medium was added. After incubation for 2.5 hours in a 37°C, 5% CO₂ incubator, 1 mL of a fresh complete medium was added for continuing culture. The cell culture supernatant was collected 48 hours after transduction. Each sample was diluted with a loading buffer under a non-reducing condition (FIG. 5b) and a reducing condition (FIG. 5c), respectively, and Western blot was performed with a goat anti-human IgG antibody (Invitrogen, Cat No: A18811) to detect the expression of ABTAA.

### B. Results

As shown in FIG. 5, both single vector transduction and dual vector transduction successfully produce ABTAA in HEK293FT cells. Diagram b shows the electrophoresis in a loading non-reducing buffer, in which the heavy and light chains of ABTAA are present in the form of a tetramer. Diagram c shows the electrophoresis in a loading reducing buffer, in which ABTAA is dissociated, and the heavy chain and the light chain are present in the form of a single chain, respectively. The expression of the antibody by dual vector transduction (lanes 2-6) is significantly higher than that by single vector transduction (lane 1). With the increase of the proportion of the light chain vector, not only the concentration of the expressed antibody increases, but also the content of non-specific bands produced by mispairing decreases.

### Example 6 Bispecific antibody expression mediated by dual rAAV vectors

In this example, dual AAV vectors are used to deliver bispecific antibodies, wherein the first antibody is ABTAA having a sequence identical to that of the ABTAA in example 5, and the second antibody is an anti-VEGF single chain antibody (VEGF scFv). The variable region of the second antibody is linked to the heavy chain or light chain of the first antibody and delivered by one AAV vector, and correspondingly, the light chain or heavy chain of the first antibody is delivered by another AAV vector.

### A. Materials and methods

1. Vector construction and viral packaging: The first combination: dual vectors ABTAA-H-G15-VEGF-LH and ABTAA-L, as shown in FIG. 6a. For vector ABTAA-H-G15-VEGF-LH, the light chain and heavy chain variable regions of an anti-VEGF antibody were linked to the heavy chain of ABTAA via a linker peptide. The gene was designed and synthesized by sequentially linking an ABTAA heavy chain leader sequence, an ABTAA heavy chain, a linker peptide and VEGF-scFv-VL-VH in series; Not I and Hind III restriction sites were added in the upstream and downstream, respectively; the gene was constructed between the promoter of an AAV expression vector pAAV.CMV.PI.EGFP.WPRE.bGH and poly A by enzyme digestion and linking; and a transfer plasmid of ABTAA-H-G15-VEGF-LH was obtained by transformation, cloning and amplification. The sequence of ABTAA was identical to that in example 5. The heavy chain and light chain variable regions of the anti-VEGF antibody were formed by splicing with reference to SEQ ID NOs: 7 and 8 in the patent US 6,884,879 B1, respectively. The target sequence alignment by Sanger sequencing showed that the inserted sequence was completely correct. The sequences used therein were as follows:

| ABTAA-H-G15-VEGF-LH | |
|---|---|
| **Name** | **Nucleotide sequence** |
| ABTAA heavy chain leader chain | ATGGGCTGGTCCTGCATCATCCTGTTCCTGGTGGCCACCGCTACCGGAG TGCACTCC (SEQ ID NO: 6) |
| ABTAA heavy chain | GACGTGCAGCTGCAAGAGTCCGGCCCTGGCCTGGTGAAGCCTTCCCA GTCCCTGTCCCTGACCTGCACCGTGACCGGCTACTCCATCACCTCCGAC TACGCCTGGAACTGGATCAGACAGTTCCCCGGCAACAAGCTGGAATGG ATGGGCTACATCAACTACTCTGGAAACACCGACTACAACCCTTCTCTGA AGTCCAGATCCTCTATCACCAGAGATACCTCCAAGAACCAGTTCTTCCT GCAGCTGAACTCCGTGACCACCGGCGACACCGCCACCTACTACTGCGC |

| | |
|---|---|
| | CAGAGGCAACTTCGAGGGCGCCATGGACTACTGGGGCCAGGGCACCT CTGTGACAGTGTCCTCCGCCTCTACAAAGGGCCCCAGCGTTTTCCCAC TGGCTCCCTGTAGCAGAAGCACCAGCGAATCTACAGCCGCTCTGGGCT GCCTGGTCAAGGACTACTTTCCTGAGCCTGTGACCGTGTCCTGGAACT CTGGCGCTCTGACAAGCGGAGTGCACACCTTTCCAGCCGTGCTGCAAA GCAGCGGCCTGTACTCTCTGAGCAGCGTGGTCACAGTGCCTAGCTCTA GCCTGGGCACCAAGACCTACACCTGTAATGTGGACCACAAGCCTAGCA ACACCAAGGTGGACAAGCGCGTGGAATCTAAGTACGGCCCTCCTTGTC CTCCATGTCCTGCACCTGAGTTTCTCGGCGGACCCTCCGTGTTCCTGTT TCCTCCAAAGCCTAAGGATACCCTGATGATCAGCAGAACCCCTGAAGT GACCTGCGTGGTGGTGGACGTGTCCCAAGAGGATCCTGAGGTGCAGTT CAATTGGTACGTGGACGGCGTGGAAGTGCACAACGCCAAGACCAAGC CTAGAGAGGAACAGTTCAACAGCACCTACAGAGTGGTGTCCGTGCTG ACAGTGCTGCACCAGGATTGGCTGAACGGCAAAGAGTACAAGTGCAA GGTGTCCAACAAGGGCCTGCCTAGCAGCATCGAGAAAACCATCAGCA AGGCCAAGGGCCAGCCAAGAGAACCCCAGGTGTACACACTGCCTCCA AGCCAAGAGGAAATGACCAAGAACCAGGTGTCCCTGACCTGCCTCGT GAAGGGATTCTACCCCTCCGATATCGCCGTGGAATGGGAGAGCAATGG ACAGCCCGAGAACAACTACAAGACAACCCCTCCTGTGCTGGACAGCG ACGGCTCATTCTTCCTGTACAGCAGACTGACCGTGGACAAGAGCAGAT GGCAAGAGGGCAACGTGTTCTCCTGCAGCGTGATGCACGAGGCCCTG CACAACCACTACACCCAGAAGTCTCTGTCCCTGTCTCTGGGCAAG (SEQ ID NO: 7) |
| Linker peptide | ggcggcggtggctccggtggaggtggatctggcggtggaggatct (SEQ ID NO: 11) |
| VEGF VL-VH (scFv) | Gatatccagctgacccagtctccttctagcttgtctgcctctgtgggcgacagagtgaccatcacctgctccgccagc caggacatctccaactacctgaactggtaccagcagaagcctggcaaggctcctaaggtgctgatctacttcacctc ctctctgcactctggagtgccctcccggttcagtggttctggctctggcaccgactttaccctgaccatctcttccctgc agcccgaggacttcgccacgtactactgccagcagtactccaccgtgccttggacctttggccagggcaccaaggt ggagatcaagggcggcggcggctctggcggcggaggctccggcggtggcggcagcgaagtgcagctggtgga atccggcggaggcctggtgcagcctggcggatctctgcggctgagctgtgccgcttctggctacgatttcacccact acggcatgaactgggtgcggcaggcccctggcaaaggcctggaatgggtcggatggatcaacacatacaccggc gagcctacctacgctgccgacttcaagcggagattcaccttcagcctggacacctctaaatctaccgcctacctgcag atgaactccctgagagccgaggataccgccgtgtactactgcgccaagtacccctactattatggcacatctcactgg tatttcgacgtgtggggccagggcaccctggtcaccgtgtcctcttga (SEQ ID NO: 12) |

The transfer plasmid of ABTAA-L was the same as the transfer plasmid of the AAV-ABTAA light chain in example 5. The sequences used therein were as follows:

| ABTAA light chain (ABTAA-L) | |
|---|---|
| **Name** | **Nucleotide sequence** |
| ABTAA light chain | ATGGGCTGGTCCTGCATCATCCTGTTCCTGGTGGCCACCGCTACCGGAG TGCACTCC (SEQ ID NO: 9) |

| | |
|---|---|
| leader sequence | |
| ABTAA light chain | TCCATCGTGATGACCCAGACCCCTAAGTTCCTGCTGGTGTCTGCTGGCG ACAGAGTGACCATCACCTGCAAGGCCTCTCAGTCCGTGTCCAACGACG TGGCCTGGTACCAGCAGAAGCCTGGCCAGTCTCCCAAGCTGCTGATCTA CTACGCCTCCAACAGATACCCCGGCGTGCCTGACCGGTTCACCGGCTCT GGCTACGGCACCGACTTCACCTTTACAATCTCCACCGTGCAGGCCGAGG ACCTGGCCGTGTACTTCTGCCAGCAGGACTACTCCTCTCCTTGGACCTT CGGCGGCGGAACCAAGCTGGAAATCAAGAGAACCGTGGCCGCCCCTAG CGTGTTCATCTTTCCTCCCTCTGACGAGCAGCTGAAATCTGGCACCGCTT CCGTGGTCTGCCTGCTGAACAACTTCTACCCCAGAGAGGCTAAGGTGCA GTGGAAAGTGGACAACGCCCTGCAGTCTGGCAACTCCCAAGAAAGCGT GACCGAGCAGGACTCCAAGGACTCTACATACAGCCTGTCCTCCACCCTG ACCCTGTCCAAGGCCGATTACGAGAAGCACAAGGTCTACGCTTGTGAA GTGACCCACCAGGGACTGTCCTCCCCTGTGACCAAGTCTTTCAATCGGG GCGAGTGCtga (SEQ ID NO: 10) |

The second combination: dual vectors ABTAA-L-E4-VEGF-LH and ABTAA-H, as shown in FIG. 6b. For vector ABTAA-L-E4-VEGF-LH, the light chain and heavy chain variable regions of an anti-VEGF antibody were linked to the light chain of ABTAA via a linker peptide. The gene was designed and synthesized by sequentially linking an ABTAA light chain leader sequence, an ABTAA light chain, a linker peptide and VEGF-scFv-VL-VH in series; Not I and Hind III restriction sites were added in the upstream and downstream, respectively; the gene was constructed between the promoter of an AAV expression vector pAAV.CMV.PI.EGFP.WPRE.bGH and poly A by enzyme digestion and linking; and a transfer plasmid of ABTAA-L-E4-VEGF-LH was obtained by transformation, cloning and amplification. The target sequence alignment by Sanger sequencing showed that the inserted sequence was completely correct. The sequences used therein were as follows:

| ABTAA-L-E4-VEGF-LH | |
|---|---|
| **Name** | **Nucleic acid sequence** |
| Light chain leader sequence | ATGGGCTGGTCCTGCATCATCCTGTTCCTGGTGGCCACCGCTACCGGA GTGCACTCC (SEQ ID NO: 9) |
| ABTAA light chain | TCCATCGTGATGACCCAGACCCCTAAGTTCCTGCTGGTGTCTGCTGGC GACAGAGTGACCATCACCTGCAAGGCCTCTCAGTCCGTGTCCAACGA CGTGGCCTGGTACCAGCAGAAGCCTGGCCAGTCTCCCAAGCTGCTGA TCTACTACGCCTCCAACAGATACCCCGGCGTGCCTGACCGGTTCACC |
| | GGCTCTGGCTACGGCACCGACTTCACCTTTACAATCTCCACCGTGCA GGCCGAGGACCTGGCCGTGTACTTCTGCCAGCAGGACTACTCCTCTC CTTGGACCTTCGGCGGCGGAACCAAGCTGGAAATCAAGAGAACCGT GGCCGCCCCTAGCGTGTTCATCTTTCCTCCCTCTGACGAGCAGCTGA AATCTGGCACCGCTTCCGTGGTCTGCCTGCTGAACAACTTCTACCCC AGAGAGGCTAAGGTGCAGTGGAAAGTGGACAACGCCCTGCAGTCTG GCAACTCCCAAGAAAGCGTGACCGAGCAGGACTCCAAGGACTCTAC ATACAGCCTGTCCTCCACCCTGACCCTGTCCAAGGCCGATTACGAGA AGCACAAGGTCTACGCTTGTGAAGTGACCCACCAGGGACTGTCCTCC CCTGTGACCAAGTCTTTCAATCGGGGCGAGTGC (SEQ ID NO: 13) |
| Linker peptide | gaatcgaagtacggacctccatctccacctagtcca (SEQ ID NO: 14) |
| VEGF VL-VH (scFv) | gatatccagctgacccagtctccttctagcttgtctgcctctgtgggcgacagagtgaccatcacctgctccgccag ccaggacatctccaactacctgaactggtaccagcagaagcctggcaaggctcctaaggtgctgatctacttcac ctcctctctgcactctggagtgccctcccggttcagtggttctggctctggcaccgactttaccctgaccatctcttcc ctgcagcccgaggacttcgccacgtactactgccagcagtactccaccgtgccttggacctttggccagggcacc aaggtggagatcaagggcggcggcggctctggcggcggaggctccggcggtggcggcagcgaagtgcagc tggtggaatccggcggaggcctggtgcagcctggcggatctctgcggctgagctgtgccgcttctggctacgatt tcacccactacggcatgaactgggtgcggcaggcccctggcaaaggcctggaatgggtcggatggatcaacac atacaccggcgagcctacctacgctgccgacttcaagcggagattcaccttcagcctggacacctctaaatctacc gcctacctgcagatgaactccctgagagccgaggataccgccgtgtactactgcgccaagtacccctactattatg gcacatctcactggtatttcgacgtgtggggccagggcaccctggtcaccgtgtcctctTGA (SEQ ID NO: 12) |

The transfer plasmid of ABTAA-H was the same as the transfer plasmid of the AAV-ABTAA heavy chain in example 5. The sequences used therein were as follows:

| **ABTAA heavy chain (ABTAA-H)** | |
|---|---|
| **Name** | **Nucleotide sequence** |
| ABTAA heavy chain leader sequence | ATGGGCTGGTCCTGCATCATCCTGTTTCTGGTGGCCACCGCTACAGGA GTGCACTCC (SEQ ID NO: 6) |
| ABTAA heavy chain | GACGTGCAGCTGCAAGAGTCCGGCCCTGGCCTGGTGAAGCCTTCCC AGTCCCTGTCCCTGACCTGCACCGTGACCGGCTACTCCATCACCTCC GACTACGCCTGGAACTGGATCAGACAGTTCCCCGGCAACAAGCTGG AATGGATGGGCTACATCAACTACTCTGGAAACACCGACTACAACCCT TCTCTGAAGTCCAGATCCTCTATCACCAGAGATACCTCCAAGAACCA GTTCTTCCTGCAGCTGAACTCCGTGACCACCGGCGACACCGCCACCT ACTACTGCGCCAGAGGCAACTTCGAGGGCGCCATGGACTACTGGGG CCAGGGCACCTCTGTGACAGTGTCCTCCGCCTCTACAAAGGGCCCCA GCGTTTTCCCACTGGCTCCCTGTAGCAGAAGCACCAGCGAATCTACA |
| | GCCGCTCTGGGCTGCCTGGTCAAGGACTACTTTCCTGAGCCTGTGAC CGTGTCCTGGAACTCTGGCGCTCTGACAAGCGGAGTGCACACCTTTC CAGCCGTGCTGCAAAGCAGCGGCCTGTACTCTCTGAGCAGCGTGGTC ACAGTGCCTAGCTCTAGCCTGGGCACCAAGACCTACACCTGTAATGT GGACCACAAGCCTAGCAACACCAAGGTGGACAAGCGCGTGGAATCT AAGTACGGCCCTCCTTGTCCTCCATGTCCTGCACCTGAGTTTCTCGGC GGACCCTCCGTGTTCCTGTTTCCTCCAAAGCCTAAGGATACCCTGATG ATCAGCAGAACCCCTGAAGTGACCTGCGTGGTGGTGGACGTGTCCCA AGAGGATCCTGAGGTGCAGTTCAATTGGTACGTGGACGGCGTGGAA GTGCACAACGCCAAGACCAAGCCTAGAGAGGAACAGTTCAACAGCA CCTACAGAGTGGTGTCCGTGCTGACAGTGCTGCACCAGGATTGGCTG AACGGCAAAGAGTACAAGTGCAAGGTGTCCAACAAGGGCCTGCCTA GCAGCATCGAGAAAACCATCAGCAAGGCCAAGGGCCAGCCAAGAGA ACCCCAGGTGTACACACTGCCTCCAAGCCAAGAGGAAATGACCAAG AACCAGGTGTCCCTGACCTGCCTCGTGAAGGGATTCTACCCCTCCGA TATCGCCGTGGAATGGGAGAGCAATGGACAGCCCGAGAACAACTAC AAGACAACCCCTCCTGTGCTGGACAGCGACGGCTCATTCTTCCTGTA CAGCAGACTGACCGTGGACAAGAGCAGATGGCAAGAGGGCAACGTG TTCTCCTGCAGCGTGATGCACGAGGCCCTGCACAACCACTACACCCA GAAGTCTCTGTCCCTGTCTCTGGGCAAGtga (SEQ ID NO: 15) |

Packaging and harvesting of the recombinant AAV were consistent with the method of example 5. pAAV.R2C2, pAdDelta6 and each of the above-mentioned transfer plasmids were co-transfected into 2E+08 HEK293FT cells in a total amount of 1040 µg DNA and a molar ratio of 1 : 1 : 1. The recombinant AAV vectors, i.e., ABTAA-H-G15-VEGF-LH, ABTAA-L, ABTAA-L-E4-VEGF-LH and ABTAA-H, were harvested 48 hours after transfection, and the viral genome titre of the crude cell lysate of each recombinant AAV vector was determined by droplet digital PCR (ddPCR).

### 2. Vector transduction and antibody detection

ABTAA-H-G15-VEGF-LH and ABTAA-L, or ABTAA-L-E4-VEGF-LH and ABTAA-H were used to co-transduce HEK293FT cells, in which the ratio of the ABTAA light chain (ABTAA-L) vector to the ABTAA heavy chain (ABTAA-H) vector was 6 : 1, 4 : 1, 2 : 1, 1:1, 1 : 2 and 1 : 4 (based on a viral genome titre), respectively, and each AAV vector also transduced cells separately. 293FT cells were seeded in a 24-well plate at 1.2E+05 cells per well and cultured overnight in a DMEM/10% FBS complete medium at 37°C under 5% CO₂. The medium was removed. A total amount of 7.0E+09 vg of a viral vector diluted with the above-mentioned complete medium was added. After incubation for 2.5 hours in a 37°C, 5% CO₂ incubator, 1 mL of a fresh complete medium was added for continuing culture. The cell culture supernatant was collected 48 hours after transduction. Each sample was diluted with a non-reducing loading buffer, respectively, and Western blot was performed with a goat anti-human IgG antibody (Invitrogen, Cat No: A18811) to detect the expression of ABTAA, wherein the positive control was purified ABTAA.

### B. Results

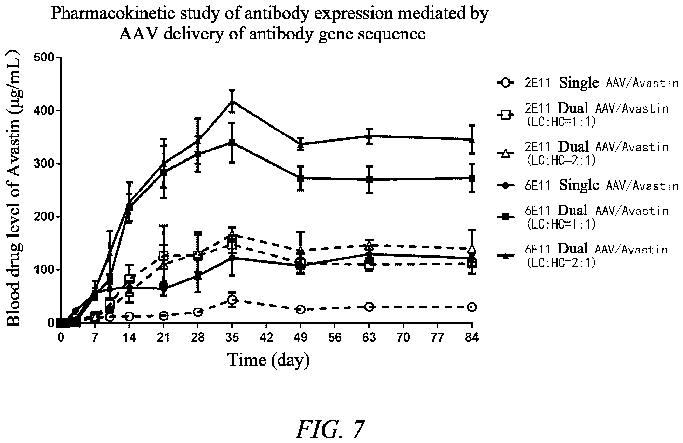

As shown in FIG. 6, both AAV vector combinations can produce an ABTAA-VEGF bispecific antibody. When the second antibody is linked to the heavy chain of the first antibody, as shown in FIG. 6c, the higher the proportion of the light chain vector, the higher the expression of the antibody, and no obvious mispairing band appears. When the second antibody is linked to the light chain of the first antibody, as shown in FIG. 6d, more non-specific bands produced by mispairing appear; and no obvious non-specific band is produced until the ratio of the light chain to the heavy chain is 6 : 1 (FIG. 7d, lane 1). The efficiency of the dual AAV vectors in expressing the single antibody ABTAA (lanes 7 and 8) is higher than that in expressing the bispecific antibody.

### Example 7 Expression of Avastin gene delivered by dual AAV vectors in mice in vivo

In this example, the dual AAV vectors are used to deliver Avastin gene into mice, and the delivery efficiency of the dual AAV vectors are compared with that of a single AAV vector. The AAV serotype was AAV8, which is the same as the vector in example 2.

### A. Materials and methods

Animal test design: eighteen SPF C57BL/6 mice were randomly divided into 6 groups with 3 mice in each group. Each mouse was injected with the corresponding total amount of the following vectors or vector combinations via a single tail vein injection at 100 µL/mouse:

| | |
|---|---|
| AAV8-Avastin | 2E+11vg |
| AAV8-Avastin | 6E+11vg |
| AAV8-Avastin -L:AAV8-Avastin-H 1 : 1 | 2E+ 11vg |
| AAV8-Avastin -L:AAV8-Avastin-H 1 : 1 | 6E+ 11vg |
| AAV8-Avastin -L:AAV8-Avastin-H 2 : 1 | 2E+ 11vg |
| AAV8-Avastin -L:AAV8-Avastin-H 2 : 1 | 6E+11vg |

Serum was collected and prepared from mice before and after administration, with the following time points for serum collection: 0d before administration to test animals; and 1d, 3d, 7d, 10d, 14d, 21d, 28d, 35d, 49d, 63d and 84d after administration. The concentration of Avastin protein in the serum sample was determined by using ELISA. The mean and SD of the blood drug level at each blood collection point were calculated. Concentration-time curve was made using GraphPad Prism 6.

### B. Results

As shown in FIG. 7, 3 days after the single AAV vector or the dual vectors enter mice, the expression of Avastin is detected, in which the blood drug levels all rise rapidly first, then decrease, and finally reach plateau, and the peak concentration appears on about 35 days after injection, and reaches plateau on about 45 days after injection. For the high-titre single vector group and the low-titre single vector group, the peak blood drug levels are 122.42 ± 33.23 µg/mL and 44.21 ± 13.50 µg/mL, respectively, and the final stable concentrations are 121.61 ± 6.96 µg/mL and 30.54 ± 6.66 µg/mL, respectively. When the ratio of the light chain to the heavy chain in the dual vectors is 1 : 1, the high-titre group and the low-titre group have peak blood drug levels of 339.53 ± 37.19 µg/mL and 148.69 ± 16.63 µg/mL, respectively, and have final stable concentrations of 272.69 ± 26.53 µg/mL and 111.84 ± 19.56 µg/mL, respectively. When the ratio of the light chain to the heavy chain in the dual vectors is 2 : 1, the high-titre group and the low-titre group have peak blood drug levels of 417.52 ± 20.35 µg/mL and 166.64 ± 14.08 µg/mL, respectively, and have final stable concentrations of 345.68 ± 26.38 µg/mL and 140.73 ± 34.34 µg/mL, respectively. The blood drug level in the high-titre group is significantly higher than that in the low-titre group, indicating a good correspondence between the titre of the injected vector and the expression of the antibody. In the same titre group, the expression by the dual vectors is higher the expression by the single vector, and when the total titre is the same but the proportion of the light chain is higher than that of the heavy chain, the expression of the antibody is further increased.

The embodiments of the present invention are not limited to the above-mentioned examples, and various modifications and improvements in forms and details may be made to the present invention by those of ordinary skill in the art without departing from the spirit and scope of the present invention, and the modifications and improvements are considered to fall within the scope of protection of the present invention.

## Claims

1. A dual recombinant viral vector system for expressing, in a cell, a polypeptide molecule which is composed of two different peptide chains and has an antigen binding activity, comprising:
a first recombinant viral vector containing, in a genome thereof, a first expression cassette for expressing the first chain of the polypeptide molecule, and
a second recombinant viral vector containing, in a genome thereof, a second expression cassette for expressing the second chain of the polypeptide molecule,
wherein the first chain and the second chain are different; the first recombinant viral and the second recombinant viral, after being introduced into a cell, can express the first chain and the second chain, respectively, and the first and second chains can be assembled into a polypeptide molecule having an antigen binding activity.

2. The dual recombinant viral vector system according to claim 1, wherein the first recombinant viral vector and the second recombinant viral vector are independently selected from the group consisting of a recombinant adeno-associated viral vector, a recombinant adenoviral vector, a recombinant HSV vector, a recombinant lentiviral vector and a recombinant retroviral vector.

3. The dual recombinant viral vector system according to claim 1 or 2, wherein the first recombinant viral vector and the second recombinant viral vector are recombinant adeno-associated viral vectors, or the first recombinant viral vector and the second recombinant viral vector are lentiviral vectors.

4. The dual recombinant viral vector system according to claim 3, wherein the first recombinant viral vector and the second recombinant viral vector are recombinant adeno-associated viral vectors, and respectively contain a capsid of AAV1, AAV2, AAV3, AAV4, AAV5, AAV6, AAV7, AAV8, AAV9, AAV10, AAV1 1, AAV12, AAV13 or AAV Rh10, or a chimeric AAV capsid or a modified capsid.

5. The dual recombinant viral vector system according to claim 3 or 4, wherein the first recombinant viral vector and the second recombinant viral vector contain an ITR of AAV1, AAV2, AAV3, AAV4, AAV5, AAV6, AAV7, AAV8, AAV9, AAV10, AAV 11, AAV12, AAV13 or AAV Rh10.

6. The dual recombinant viral vector system according to any one of claims 1-5, wherein the polypeptide molecule contains an antigen binding moiety having a heavy chain variable region and a light chain variable region, the first chain contains the heavy chain variable region of the antigen binding moiety, and the second chain contains the light chain variable region of the antigen binding moiety.

7. The dual recombinant viral vector system according to claim 6, wherein the polypeptide molecule contains an antigen binding moiety having a heavy chain and a light chain, the first chain contains the heavy chain of the antigen binding moiety, and the second chain contains the light chain of the antigen binding moiety.

8. The dual recombinant viral vector system according to claim 6 or 7, wherein a ratio of the first recombinant viral vector to the second recombinant viral vector is 4 : 1-1 : 8, preferably 1 : 1-1 : 4.

9. The dual recombinant viral vector system according to any one of claims 6-8, wherein the polypeptide molecule is a monoclonal antibody or an antigen binding fragment thereof, the first chain is a heavy chain of the monoclonal antibody or the antigen binding fragment thereof, and the second chain is a light chain of the monoclonal antibody or the antigen binding fragment thereof.

10. The dual recombinant viral vector system according to claim 9, wherein the antigen binding fragment is an Fab fragment, an Fab' fragment or a (Fab')₂ fragment.

11. The dual recombinant viral vector system according to claim 9 or 10, wherein the monoclonal antibody or the antigen binding fragment thereof specifically binds to an angiogenic factor.

12. The dual recombinant viral vector system according to claim 11, wherein the angiogenic factor is selected from VEGF, VEGFR, FGF, PDGF, TGFβ, Ang1, Ang2, integrin, CD31 and a plasminogen activator.

13. The dual recombinant viral vector system according to any one of claims 6-8, wherein the polypeptide molecule is a bispecific antibody comprising a first antigen binding moiety that specifically binds to a first antigenic epitope and a second antigen binding moiety that specifically binds to a second antigenic epitope, the first antigenic epitope and the second antigenic epitope are different; the first antigen binding moiety comprises a heavy chain and a light chain, the first chain contains the heavy chain of the first antigen binding moiety, and the second chain contains the light chain of the first antigen binding moiety.

14. The dual recombinant viral vector system according to claim 13, wherein the first antigen binding moiety is a monoclonal antibody or an antigen binding fragment thereof, preferably the antigen binding fragment is an Fab fragment, an Fab' fragment or a (Fab')₂ fragment.

15. The dual recombinant viral vector system according to claim 13 or 14, wherein the second antigen binding moiety is an antibody in the form of a single chain.

16. The dual recombinant viral vector system according to claim 15, wherein the antibody in the form of a single chain is a single domain antibody or scFv.

17. The dual recombinant viral vector system according to claim 15 or 16, wherein the second antigen binding moiety is linked to the N-terminus or C-terminus of the heavy chain of the first antigen binding moiety; and the first chain contains the heavy chain of the first antigen binding moiety, and the second antigen binding moiety.

18. The dual recombinant viral vector system according to any one of claims 15-17, wherein the second antigen binding moiety is linked to the N-terminus or C-terminus of the light chain of the first antigen binding moiety; and the second chain contains the light chain of the first antigen binding moiety, and the second antigen binding moiety.

19. The dual viral vector system according to any one of claims 15-18, wherein the first chain contains the heavy chain of the first antigen binding moiety, and the second antigen binding moiety; and the second chain contains the light chain of the first antigen binding moiety.

20. The dual recombinant viral vector system according to any one of claims 15-19, wherein at least one or both of the first antigen binding moiety and the second antigen binding moiety specifically bind to an angiogenic factor.

21. The dual recombinant viral vector system according to claim 20, wherein the angiogenic factor is selected from VEGF, VEGFR, FGF, PDGF, TGFβ, Ang1, Ang2, integrin, CD31 and a plasminogen activator.

22. The dual recombinant viral vector system according to claim 20, wherein the first antigen binding moiety specifically binds to VEGF, and the second antigen binding moiety specifically binds to Ang2; or the first antigen binding moiety specifically binds to Ang2, and the second antigen binding moiety specifically binds to VEGF.

23. The dual recombinant viral vector system according to any one of claims 6-8, wherein the polypeptide molecule contains a first antigen binding moiety that specifically binds to a first antigenic epitope and a second binding domain that specifically binds to a second target molecule, the second target molecule is a cytokine, the first antigen binding moiety comprises a heavy chain and a light chain, the first chain contains the heavy chain of the first antigen binding moiety, and the second chain contains the light chain of the first antigen binding moiety.

24. The dual recombinant viral vector system according to claim 23, wherein the second binding domain is a cytokine receptor, an extracellular domain thereof, or their variants.

25. The dual recombinant viral vector system according to claim 23 or 24, wherein the first antigen binding moiety is a monoclonal antibody or an antigen binding fragment thereof, preferably the antigen binding fragment is an Fab fragment, an Fab' fragment or a (Fab')₂ fragment.

26. The dual recombinant viral vector system according to any one of claims 23-25, wherein the second binding domain is linked to the N-terminus or C-terminus of the heavy chain of the first antigen binding moiety; and the first chain contains the heavy chain of the first antigen binding moiety, and the second binding domain.

27. The dual recombinant viral vector system according to any one of claims 23-26, wherein the second binding domain is linked to the N-terminus or C-terminus of the light chain of the first antigen binding moiety; and the second chain contains the light chain of the first antigen binding moiety, and the second binding domain.

28. The dual viral vector system according to any one of claims 23-27, wherein the first chain contains the heavy chain of the first antigen binding moiety, and the second binding domain; and the second chain contains the light chain of the first antigen binding moiety.

29. The dual recombinant viral vector system according to any one of claims 20-27, wherein the first antigen binding moiety specifically targets and binds to PD-1, PD-L1 or an angiogenic factor, and the second binding domain is a receptor or a fragment thereof capable of binding to human transforming growth factor β (TGFβ), Ang2 or VEGF.

30. The dual recombinant viral vector system according to claim 29, wherein the angiogenic factor is selected from VEGF, VEGFR, FGF, PDGF, TGFβ, Ang1, Ang2, integrin, CD31 and a plasminogen activator.

31. The dual recombinant viral vector system according to claim 29, wherein the first antigen binding moiety specifically binds to PD-L1, and the second binding domain is a human transforming growth factor β receptor II (TGFβRII) or a fragment thereof.

32. The dual recombinant viral vector system according to any one of claims 1-31, wherein the system is a composition containing the first recombinant viral vector and the second recombinant viral vector.

33. A dual plasmid system for producing the dual recombinant viral vector system according to any one of claims 1-32, comprising:
a first plasmid containing the first expression cassette and a viral genomic element required for the production of the first recombinant viral vector; and
a second plasmid containing the second expression cassette and a viral genomic element required for the production of the second recombinant viral vector.

34. A host cell transduced by using the dual recombinant viral vector system according to any one of claims 1-32.

35. A pharmaceutical composition containing the dual recombinant viral vector system according to any one of claims 1-32 or the cell according to claim 34 and a pharmaceutically acceptable carrier.

36. Use of the dual recombinant viral vector system according to any one of claims 1-32, the dual plasmid system according to claim 33 or the cell according to claim 34 in the preparation of a polypeptide molecule which is composed of two different peptide chains and has an antigen binding activity.

37. A method for producing a polypeptide molecule which is composed of two different peptide chains and has an antigen binding activity, comprising the following steps:
transducing a host cell by using the dual recombinant viral vector system according to any one of claims 1-32;
and culturing the transduced host cell under conditions that enable the first chain and second chain of the polypeptide molecule to be expressed in the host cell and assembled to produce a polypeptide molecule having an antigen binding activity, thereby obtaining the polypeptide molecule.

38. Use of the dual recombinant viral vector system according to any one of claims 1-32 in the preparation of a drug for delivering a polypeptide molecule which is composed of two different peptide chains and has an antigen binding activity to a subject.

39. A method for delivering a polypeptide molecule which is composed of two different peptide chains and has an antigen binding activity to a subject, comprising administering to a subject in need thereof the dual recombinant viral vector system according to any one of claims 1-32 or the pharmaceutical composition according to claim 34.

40. The dual recombinant viral vector system according to any one of claims 1-32 or the pharmaceutical composition according to claim 34, for use in delivering a polypeptide molecule which is composed of two different peptide chains and has an antigen binding activity to a subject.
